# EUROPEAN PATENT SPECIFICATION

(11) **EP 2 114 462 B1**
(45) Date of publication and mention of the grant of the patent: **05.06.2013**
(21) Application number: 07870433.5
(22) Date of filing: 21.12.2007
(51) Int. Cl.: A61K 49/00

(54) **Compounds for fluorescence imaging**
Verbindungen zur Fluoreszenz-Bildgebung
Composés destinés à des applications d'imagerie par fluorescence

(30) Priority: 21.12.2006 US 871330 P
(43) Date of publication of application: 11.11.2009
(73) Proprietor: UNIVERSITE DE GENEVE, 1211 Geneve 4 (CH)
(72) Inventor: LANGE, Norbert, CH-1260 Nyon (CH); CAMPO, Marino, A., CH-4500 Solothurn (CH)
(74) Representative: Vossius & Partner
(86) International application number: PCT/IB2007/004385
(87) International publication number: WO 2008/078190

(56) References cited:
- WO-A-2007/023398
- US-A1- 2006 275 775
- TUNG C-H ET AL: "PREPARATION OF A CATHEPSIN D SENSITIVE NEAR-INFRARED FLUORESCENCE PROBE FOR IMAGING" BIOCONJUGATE CHEMISTRY, ACS, WASHINGTON, DC, US, vol. 10, 1 September 1999 (1999-09-01), pages 892-896, XP001222045 ISSN: 1043-1802 cited in the application
- FUNOVICS M. ET AL.: "Protease sensors for bioimaging" ANALYTICAL AND BIOANALYTICAL CHEMISTRY, vol. 377, no. 6, September 2003 (2003-09), pages 956-963, XP002484451 cited in the application
- GABRIEL D. ET AL.: "Tailoring protease-sensitive photodynamic agents to specific disease-associated enzymes" BIOCONJUGATE CHEMISTRY, vol. 18, no. 4, July 2007 (2007-07), pages 1070-1077, XP002484452

## Description

### 1. Field of the Invention

The present invention relates generally to the fields of fluorescence imaging, polymer chemistry, peptide chemistry, cell biology, and diagnostic imaging. More particularly, it concerns enzyme-activatible fluorescent imaging probes.

### 2. Description of Related Art

Significant limitations exist in conventional imaging techniques. Due to limitations in differentiating between normal and diseased tissue, conventional imaging techniques (*e.g.*, anatomical, structural, and endoscopical approaches) often fail to provide the essential information vital for the proper diagnosis and management of cancer patients. Many aggressive internal tumors containing as few as 500000 cells (∼2mm in diameter) are likely to pass undetected through most body-region scans, including CT, MRI, ultrasound, radionucleotide, and metabolic PET imaging. These imaging techniques typically require bulk tissular features. Therefore, the resulting signal is epiphenomenal, being merely an expression of the sum of non-specific interactions of the imaging device with the tissue's structure, physiology and/or pathology. The degree to which a specific tumor can be visualized by these conventional methods is merely a function of the tumor's tendency to differentially scatter, absorb, or emit radiation as compared to the healthy neighboring tissue. Additionally, such modem imaging techniques are often cost intensive and time consuming. Furthermore, the interpretation of the resulting images is often ambiguous and often requires extensive experience for proper interpretation by a physician.

There exists a need in modern oncology for sensitive and disease-specific methodologies that can deliver high resolution images online. In this context, optical methods hold great promise for such imaging purposes. While the lower detection limit for tumors in human subjects is currently at about 500000 cells, optical methods have already been shown to reveal as little as approximately 1000 cells in animal models. Fluorescence imaging is one of such optical methods, which is characterized by high resolution and sensitivity. The particular characteristics of fluorescence-based imaging have demonstrated the significant potential of this technology. Fluorescence imaging can be used both in the clinic and in preclinical studies. A major advantage of this technique is that it is minimally invasive, allowing repeated imaging of animals without euthanasia. The use of more than one fluorescent reporter or imaging technique may provide increased specificity for the diagnosis of various disease states.

Reporting technologies for molecular imaging can be broadly classified as "direct" or "indirect" depending on whether the fluorescent molecule requires no biosynthesis (direct) or must be biosynthesized (indirect). Direct technologies are based on the administration of a probe that is either fluorescently active or activatable. More specifically, activatable probes display little or no fluorescence in their native state but become activated and fluoresce only after the intended molecular event to be reported has occurred. Such events include changes in probe conformation by binding to complementary RNA sequences, as is the case for molecular beacons or, alternatively, specific enzymatic digestion of the activatable probe. These events ultimately result in a distance increase between fluorophore-quencher pairs (molecular beacons and activatable probes) and/or closely spaced fluorophores (activatable probes), which situate the fluorophores beyond the critical value required for efficient energy transfer, thus leading to an increase in fluorescence quantum yield upon light illumination.

It is currently well accepted that some diseases are associated with the over-expression of certain enzymes, including proteases. This has led Weissleder and coworkers to develop a reporter imaging technology based on protease-activatable, self-quenched probes. This concept is based on the spontaneous or enzymatic transformation of a compound from a fluorescently inactive to a fluorescently active state, which can be exploited to generate images demarcating neoplastic tissue. In one of these approaches several fluorescent reporter probes are coupled via a sequence specific peptide to a polymeric backbone (carrier). Ideally, each carrier contains a sufficient number of fluorescent reporters to allow for efficient (radiationless) energy transfer between each reporter molecule when excited with light. Therefore, due to the neighboring proximity of the fluorophores, the whole entity becomes essentially non-flourescent by autoquenching, and thus, it is "dormant."

When digested by specific proteolytic enzymes, fluorophores are released from the conjugate, allowing the fluorescent reporters to increase their mutual distance. Under these conditions, deactivation by fluorescence of the excited fluorophore becomes dominant over radiationless deactivation, thus resulting in a more intense fluorescence signal at sites with increased amounts of the targeted enzymatic activity. Weissleder and coworkers have elegantly illustrated this idea by showing how the targeting of oncologically related enzymes, in particular cathepsins, with such probes can lead to high quality images displaying good selectivity and sensitivity for neoplasia in experimental tumor models. In a first generation of such fluorescent probes, Weissleder and coworkers prepared a long circulating polylysine-PEG conjugate, in which a number of the fluorophore Cy 5.5 were directly tethered to the free amino groups of the polylysine backbone. In this earlier case, activation of the probe was accomplished by the enzymatic degradation of the polymer backbone by either cathepsin B or trypsin. In a later probe design, Weissleder and colleagues used a polylysine-PEG copolymer bearing 11 to 55 Cy 5.5 fluorophores, which were covalently tethered by cathepsin D cleavable linkers with the following sequence Gly-Pro-Ile-Cys(Et)-Phe-Phe-Arg-Leu-Gly. After i.v. injection of this probe to mice bearing a BT-20 mammary adenocarcinoma, the tumors became brightly fluorescent within 12-48 hours, attaining a maximum at 24 hours post administration. T/N ratios were highest for tumor/muscle (∼120) and lowest for tumor/blood (∼11).

However, certain problems have been associated with this specific approach. For example, T/N fluorescence ratios as low as 2 have been reported, which can be attributed to the high intrinsic fluorescence of these probes in their dormant state and limited access of enzymes to the site of cleavage. Therefore, in order to further improve this methodology it is essential to further decrease the fluorescence quantum yield of the non-digested probe.

Most of the work done by Weissleder and coworkers is based on the use of a poly-L-lysine backbone, which is easily degraded in a non-specific fashion by proteases *in vivo.* In order to improve backbone stability and biocompatibility, the "free" residual poly-L-lysine side chains were protected by derivatizing them with polyethylene glycol chains. It has also been suggested in publications by the author that backbone protection can also be accomplished by tethering a variety of poly- and oligosaccharides as well as many other natural and synthetic polymers (Funovics *et al.,* 2003; Zhou *et al.,* 2003; Mahmood and Weissleder, 2003; Pham *et al.,* 2004; WO 200308; WO 2002056670; WO 2002000265; WO 9958161). Other polymer quenched probes have been also reported for the imaging of protease activity (see McIntyre *et al.,* 2004; Bigelow *et al.,* 2004), and certain compounds comprising the polymer polylysinc have been used for imaging (Funovics *et al.,* 2003, Zhou *et al.,* 2003; Mahmood, and Weissleder, 2003; Pham *et al.,* 2004; WO 2003082988; WO 2002056670; WO 2002000265; WO 9958161; McIntyre et al., 2004; US Patent 6,592,847; US Application 2006/0275775). Unfortunately, PEG-polylysine graft polymers display the undesirable quality of being significantly more fluorescent than corresponding probes lacking PEG chains, and the desired specific enzymatic activation of these probes appears to be greatly inhibited by the graft architecture of the polymer. Clearly, there exists a need for improved compositions and methods for selective imaging of diseased and/or target tissues.

### SUMMARY OF THE INVENTION

The present invention overcomes limitations in the prior art by providing imaging agents according to the claims with substantially improved properties (*e.g*., improved self-quenching in the absence of enzyme activation and/or reduced diffusion of enzyme-activated imaging agents from the site of activation); also provided are improved methods for synthesizing imaging probes. The present invention is based, at least in part, on the surprising observation that conjugation of a water-solubilizing moiety to an enzyme-activatible polymer carrying fluorophores results in a substantial improvement in the characteristics of the imaging agent (*e.g*., decreased fluorescence in the absence of enzymatic activation and thus increased resolution of fluorescence as a result of enzymatic activation). Without wishing to be bound by any theory, the inventors believe that hydrophilic water solubilizing moieties interact in conjunction with hydrophobic fluorophores to result in conformations which bring the fluorophores into close proximity and enhances quenching or self quenching; accordingly, enzymatic degradation of the imaging probe would allow for release of the fluorophore from the imaging agent and an increase in fluorescence at least partly due to decreased quenching or self-quenching. In certain embodiments, imaging agents of the present invention possess improved characteristics (e.g., reduced fluorescence in the absence of enzymatic activation) as compared to commonly used PEG-polylysine graft polymers carrying different amounts of fluorophorores.

One aspect of the invention involves conjugating a small charged moiety, such as a quaternized ammonium salt (*e.g.,* a 1-N-methylnicotinic group), to a polymer backbone (*e.g*., polylysine). In various embodiments, the small charged moiety may be a pyridyl-substituted-acyl (*e.g*., wherein the pyridyl nitrogen is quaternized or alkylated), a heteroaryl-substituted-acyl, or a pyridinal. This approach displays significant advantages over the sole conjugation of PEG to an imaging agent. Without wishing to be bound by any theory, the data provided in the below examples support the idea that these new conjugates not only show substantially improved fluorescence quenching as compared to their native counterparts, but also significantly higher quenching efficacy as their PEGylated counterparts, by maintaining good water solubility. In addition, the below examples illustrate that, in contrast to their PEGylated counterparts, certain small moieties do not impede access of enzymes to the specific cleavage sites, making activation of the new probe much more efficient in the presence of the pathological proteolytic enzyme. Similarly, using small hydrophilic moieties such as simple modified sugar derivatives also produced a similar effect, although this effect was not quite as dramatic as was observed with 1-methylnicotinic acid. In certain embodiments, small charged moieties or small hydrophilic moieties may be used to partially or completely protect the polymeric backbone from non-specific enzymatic degradation.

The present invention further provides imaging probes according to the claims comprising a hydrophilic polymer conjugated to both small charged/hydrophilic moieties and lipophilic fluorophores. This amphiphilic probe architecture has proven to be advantageous over non-amphiphilic architectures in terms of fluorescent quenching/activation. The inventors were surprised to find that probe quenching/activation behavior was optimal for amphiphilic probes in which the polymer backbone and side chains are hydrophilic while the fluorophore is lipophilic. Without being bound to any theory, this structural motif allows for the fluorophores to aggregate intramolccularly in aqueous media giving rise to better energy transfer and quenching. The inventor also observed that *in vivo* enzymatic activation of these amphiphilic probes released liphophilic fluorophores at the pathological site where they remained for an increased period of time due to reduced clearance following activation. This property enhances the contrast and boundaries between pathological and healthy tissue since the fluorophore does not "leak out" from the site of interest where cleavage/activation occurs. This advantage was not observed for probes carrying hydrophilic fluorophores, which displayed shorter clearance times from the site of activation/release and quickly became diffused throughout the tissues.

Also disclosed herein are methods for imaging probe synthesis that utilize the unique advantages of the chemical structure of imaging probes of the present invention. Specifically, this amphiphilic probe architecture composed of lipophilic fluorophores and, optionally, charged moieties may be utilized in purification methods such as solid phase probe extraction and/or ion exchange purification. These methods take advantage of the high affinity for amphiphilic counter charged phases/surfaces of these probes. This enables for pH dependent or independent "polyplex" ion pairing together with liphophilic interactions between the probe and the extraction phase lipophilic components. Extraction phases include polystyrene-sulfonic acid resins, polystyrene-amine resins, polystyrene-divinylbenzene-sulfonic acid resins, polystyrene-divinylbenzene-amine resins, methacrylic-divinylbenzene resins, acrylic-divinylbenzene resins, ion-exchange resins. Thus, the probe can be easily purified after the required set of sequential chemical reactions by performing a solid phase extraction at the proper pH, then rinsing off impurities and subsequently by desorption/release of the pure probe from the solid phase by either inducing a pH change and/or increasing the ionic strength, and/or extracting with a different solvent including ionic liquids.

These synthetic strategies allow for simple and efficient probe assembly chemistry characterized by high probe yields, multi-one pot reactions and fewer purification steps. In contrast, the previously described probe assembly procedures by Weissleder and coworkers typically require long and tedious experimental procedures and multiple purification steps. Furthermore, the chemistry described by Weissleder and coworkers has the disadvantage of using iodoacetyl and other highly electrophilic moieties required for chemoselective coupling of peptides to the polymer backbone, which can produce unstable probes with a high tendency to crosslink becoming insoluble during manipulation. However, methods described herein allow for the synthesis of probes with excellent storage stability and little or no crosslinking. This chemical simplicity has allowed for the development of a simple and straight forward protocol that may be used in probe preparation kits.

An aspect of the present invention relates to an imaging probe according to the claims comprising a biocompatible hydrophilic polymer backbone conjugated to a plurality of hydrophobic fluorophores and a plurality of hydrophilic solubilizing moieties; wherein each solubilizing moiety is independently either: 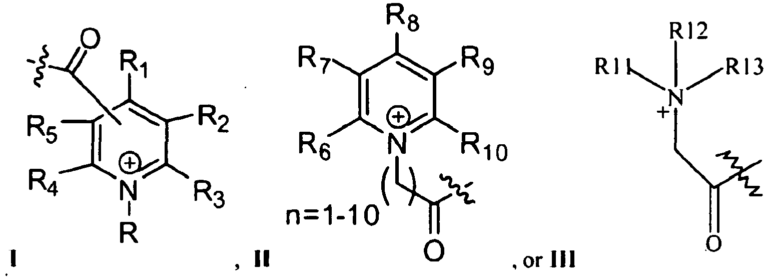 wherein R is independently hydrogen, a heteroatom-substituted or heteroatom-unsubstituted alkyl_{(C1-C10)}, aryl(_{C1-C10)}, or aralkyl_{(C2-C10)}; further wherein R₁, R₂, R₃, R₄ and R₅ are each independently hydrogen, hydroxy, amino, cyano, halo, nitro, mercapto, or a heteroatom-substituted or heteroatom-unsubstituted alkyl(_{C1-C10)}, aryl_{(C1-C10)}, aralkyl_{(C2-C10)}, acyl_{(C1-C10)}, alkoxy_{(C1-C10)}, aryloxy_{(C1-C10)}, aralkoxy_{(C2-C10)}, acyloxy_{(C1-C10)}, alkylamino_{(C1-C10)}, arylamino_{(C1-C10)}, aralkylamino_{(C2-C10)}, amido_{(C1-C10)}, or the carbonyl group that conjugates to the hydrophilic polymer backbone, provided that one of R₁, R₂, R₃, R₄ and R₅ is said carbonyl group that conjugates to the hydrophilic polymer backbone; further wherein R₆, R₇, R₈, R₉ and R₁₀ are each independently hydrogen, hydroxy, amino, cyano, halo, nitro, mercapto, or a heteroatom-substituted or heteroatom-unsubstituted alkyl(_{C1-C10}), aryl(_{C1-C10}), aralkyl(_{C1-C10}), acyl(_{C1-C10}), alkoxy(_{C1-C10}), aryloxy(_{C1-C10}), aralkoxy(_{C2-C10}), acyloxy(_{C1-C10}), alkylamino(_{C1-C10}), arylamino(_{C1-C10}), aralkylamino(_{C2-C10}), or amido(_{C1-C10}); further wherein R₁₁, R₁₂, and R₁₃, are each independently a heteroatom-substituted or heteroatom-unsubstituted alkyl_{(C1-C10)}, aryl_{(C1-C10)}, or aralkyl_{(C2-C10)}; and further wherein at least some of said fluorophores are in fluorescence-quenching or self-quenching permissive positions which are separable by enzymatic cleavage.

The fluorophores comprise a near-infrared fluorophore, 1-pyrenebutyric acid (pyrene), NIR-667, NIR-641 N-succinimidyl ester, 11-((5-dimethylaminonaphhalene-1-sulfonyl)amino)undecanoic acid, cis-parinaric acid, BODIPY® 581/591, BODIPY® FL (505/511), BODIPY® 530/550, BODIPY (C1-BODIPY 500/510 C12, C4-BODIPY 500/510 C9, C8-BODIPY 500/510 C5, a distyryl-boradiazaindacene, Dansyl (336/517), Marina Blue®, M12652 Marina Blue®, pacific blue TM, NBD (463/536), fluorescein (496/519), Oregon green® 488 (501/526), tetrametylrhodamine (540/566), lissamine rhodamine (560/581), lissamine rhodamine B sulfonyl chloride, texas red® (582/601), dansyl undecanoic acid, 6-(N-(7-nitrobenz-2-oxa-1,3-diazol-4-yl)amino)hexanoic acid, (4-fluoro-7-nitrobenz-2-oxa-1,3-diazole), (1-dodecyl,1'-(4-butyric acid NHS)-3,3,3',3'-tetramethylindocarbocyanine halide), (N-Ethyl-N-[5-(N-succininmidyloxycarbonyl)pentyl]-3, 3, 3, 3-tetramethyl-2), 2-indocarbocyanine chloride, 11-Chloro-1,1'-dipropyl-3,3,3',3'-tetramethyl-10,12-trimethyleneindatricarbocyanine iodide, NIR-664-N-succinimidyl ester, 2-[(E)-2-(2-chloro-3-{(E)-2-[3-ethyl-5-phenyl-1,3-benzoxazol-2(3H)-ylidene]ethylidene}-1-cyclopenten-1-yl)ethenyl]-3-ethyl-5-phenyl-1,3-benzoxazol-3-ium iodide, or 2-{2-chloro-3-[3-ethyl-1,3-benzothiazol-2(3H)-ylidene]-1-cyclopenten-1-yl}-3-ethyl-1,3-benzothiazol-3-ium perchlorate.

One or more of said fluorophores may conjugated to the polymer backbone via an enzyme-cleavable linker, and wherein cleavage of the enzyme-cleavable linker results in the release of at least one of said fluorophores from the polymer backbone. The enzyme-cleavable linker may be a cathepsin D cleavable linker. In certain embodiments, the cathepsin D cleavable linker comprises Gly-Pro-Ile-Cys(Et)-Phe-Phe-Arg-Leu-Gly.

In certain embodiments, wherein the solubilizing moieties each comprise a quarternary ammonium species and have a molecular weight of less than about 1 kDa. The solubilizing moieties may comprise a solubilizing moiety selected from the group consisting of: 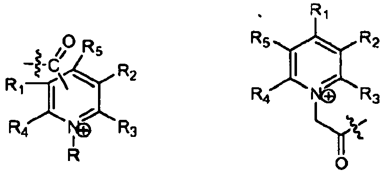 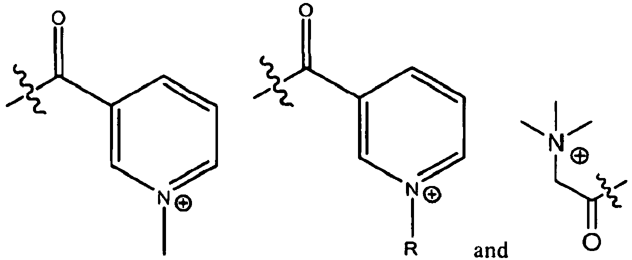 wherein R, R₁, R₂, R₃, R₄, R₅ are each independently selected from the group consisting of H, hydroxy, amino, cyano, halo, nitro, mercapto, or a heteroatom-substituted or heteroatom-unsubstituted alkyl(_{C1-C10}), aryl(_{C1-C10}), aralkyl(_{C2-C10}), acyl(_{C1-C10}), alkoxy(_{C1-C10}), aryloxy(_{C1-C10}), aralkoxy(_{C2-C10}), acyloxy(_{C1-C10}), alkylamino(_{C1-C10}), arylamino(_{C1-C10}), aralkylamino(_{C2-C10}) or amido(_{C1-C10}), or the carbonyl group that conjugates to the hydrophilic polymer backbone as indicated above. The solubilizing moiety may be a nicotinic moiety, and, in certain embodiments, R is -CH₃.

In certain embodiments, one or more of the above solubilizing compounds are chemically reacted with an amine group present in a polymer backbone (*e.g*., polylysine) to result in a solubilizing moiety covalently conjugated to the polymer backbone. Specifically, in certain embodiments, the amide bond may be formed from the reaction between a carboxylic acid residue on a solubilizing moiety and an -NH₂ group present on a polymer backbone such as polylysine. For example, the following solubilizing compounds may be chemically reacted with a polymer: 1-methylnicotinic acid, an N-methylnicotinic acid, a N-alkyl-substituted-nicotinic acid wherein the alkyl is a C₁-C₁₂ or a C₁-₆ alkyl, or a nitrosubstituted N-alkyl nicotinic acid wherein the alkyl is a C₁-C₁₂ or a C₁-C₆ alkyl, N-methyl-4-carboxypyridine, an N-methyl-2-carboxypyridine, an N-alkylcarboxysubstitutedpyridine wherein the alkyl is a C₁-C₆ alkyl,

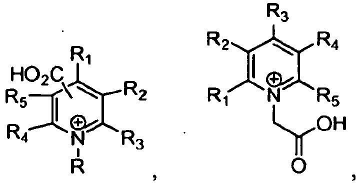

R, R₁, R₂, R₃, R₄, R₅ may be selected from the group consisting of H, hydroxy, amino, cyano, halo, nitro, mercapto, or a heteroatom-substituted or heteroatom-unsubstituted alkyl(_{C1-C10}), aryl(_{C1-C10}), aralkyl(_{C2-C10}), acyl(_{C1-C10}), alkoxy(_{C1-C10}), aryloxy(_{C1-C10}), aralkoxy(_{C2-C10}), acyloxy(_{C1-C10}), alkylamino(_{C1-C10}), arylamino(_{C1-C10}), aralkylamino(_{C2-C10}) or amido(_{C1-C10}); a substituted N-methylbenzoquinoline, a substituted N-methylacridine, a substituted N-methyl isoquinoline, a substituted N-methylphenanthredine, a substituted N-alkylated pyridine wherein the alkylated positions are C₁-C₆ alkyl moieties, an N-C₁₋₆ alkanoic acid substituted-benzopyridine, or an N-C₁₋₆ alkanoic acid substituted-dibenzopyridine. As stated above, the solubilizing compound may be a N-substituted benzopyridine or an N-substituted dibenzopyridine, such as the abovementioned 1-Alkyl-X-carboxyquinoline or 1-alkyl-X-carboxydibenzopyridine, respectively.

In certain embodiments, one or more of the above solubilizing compounds is 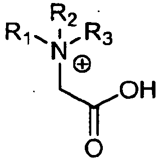 wherein R, R₁, R₂. R₃ may be a heteroatom-substituted or heteroatom-unsubstituted alkyl_{(C1-C10)}, aryl_{(C1-C10)}, or aralkyl_{(C2-C10)}.

The polymer backbone may be a peptide, a protein, a polysaccharide, a polyester, or chitosan. For example, the polymer backbone may comprise D- or L- amino acids. In certain embodiments, the polymer backbone is poly-L-lysine. The polymer backbone may be selected from the group consisting of poly-D-lysine, polyarginine, polyomitine, polyglutamic acid, polyvinyl alcohol, polyacrylic acid, polymethacrylate, polyacrylamide, polyalkylcyanoacrylate, polyhydroxyacrylate, polysuccinimide, polysuccinic anhydride, poly(hydroxyethyl methacrylate) (HEMA), a polysaccharide, dextran, aminodextran, a modified dextran, amdex®, an oligonucleotide, chitosan, trimethyl chitosan, and derivatized chitosan. The fluorophore may be conjugated to the polymer via an amide bond.

The enzyme-activatable increase in fluorescence may be caused by clevage of a bond in said imaging probe by an enzyme, wherein the enzyme is trypsin, a cathespin, cathespin D, cathespin B, cathespin H, urokinase, thrombin, plasmin, a matrix metalloproteinase (MMP), a kallikrein, a human kallikrein, a human kallikrein 3, a caspase, a calpain, a granzyme B.

The imaging probe may further comprise an enzyme-cleavable linker, wherein the enzyme-cleavable linker is a peptide, a polyester, a polysaccharide, an oligonucleotide, a synthetic polymer or monomer, or a natural polymer or monomer. Cleavage of said enzyme-cleavable linker may result in the release of the fluorophore from the imaging probe. Alternately, said cleavage may result in the release of a quencher from the imaging probe. The imaging probe may comprise the trypsin sensitive peptide linker Gly-Ala-Ser-(L)Arg-Thr-Gly.

The imaging probe may further comprise a quencher. The quencher may be in sufficient proximity to the fluorophore to reduce the fluorescence of the fluorophore. In certain embodiments, fluorophores are covalently attached to from about 0.1% to about 80 %, or from about 3% to about 50%, of the available functionalities of said polymer backbone.

The imaging probe may further comprise a targeting moiety. The targeting moiety may be an antibody or antibody fragment. The imaging probe may further comprise a biocompatibilizing unit.

In certain embodiments, the imaging probe is comprised in a pharmaceutically acceptable excipient. The pharmaceutically acceptable excipient may be formulated for intravenous, intraperitoneal, or topical administration. The imaging probe may be comprised in a kit or a container means. In certain embodiments, wherein the imaging probe has a molecular weight of from about 1 kDa to about 100,000 kDa.

An aspect of the present invention relates to an imaging probe according to the claims for use in a method of *in vivo* photodetection or imaging comprising administering the imaging probe to a subject, allowing a period of time to pass sufficient to allow for enzymatic activation of the imaging probe, and detecting the fluorescence of the fluorophores. The subject may be a mammal, such as a human. The method may comprise a method for screening a putative protease inhibitor. In certain embodiments, the method comprises a method of diagnosing or treating a disease. The disease may be a cell proliferative disease. The cell proliferative disease may be cancer, such as a breast cancer. In various embodiments, wherein the disease may be an inflammatory disease, rheumathoid arthritis, a circulatory disease, atherosclerosis, a digestive disorder, ulcers, colon polyps, or is characterized by an activation of a proteolytic enzyme.

The method may comprise irradiation of part or all of the subject with light of a wavelength corresponding to an absorption wavelength of the fluorophore. The wavelength may be between from about 350 to about 1400 nm. The step of said irradiation may be performed within a time interval of 4 minutes to 168 hours after administration of the imaging agent.

Another aspect of.the present invention relates to a method of *in vitro* photodetection or imaging comprising administering an imaging probe of the present invention to a sample, allowing a period of time to pass sufficient to allow for enzymatic activation of the imaging probe, and detecting the fluorescence of the fluorophores.

Also disclosed herein is a method of synthesizing an imaging probe according to the claims comprising: solid phase synthesis of an enzyme specific protein or pepide cleavable linker, chemoselective conjugation of a fluorophore to the enzyme specific protein or peptide cleavable linker; conjugation of the fluorophore to the polymer backbone; protection of the polymer backbone from non-specific enzymatic degradation by the use of protecting moieties on available polymer functionalities; and solid phase probe extraction or ion exchange purification or size exclusion chromatography of the imaging probe; wherein iodoacylation is not required to generate the imaging probe. The method may comprise purifying the imaging probe using an extraction phase, wherein the extraction phase comprises a polystyrene-sulfonic acid resin, a polystyrene-amine resin, a polystyrene-divinylbenzene-sulfonic acid resin, a polystyrene-divinylbenzene-amine resin, a methacrylic-divinylbenzene resin, an acrylic-divinylbenzene resin, or an ion-exchange resin. In certain embodiments, wherein the method further comprises conjugating a biocompatibilizing moiety or a solubilizing moiety or a quenching moiety to the polymer backbone. The imaging probe may be purified via the following sequential steps: solid phase extraction of the imaging probe at the proper pH; rinsing impurities away from the imaging probe; and desorption or release of the purified probe from the solid phase by: inducing a pH change and/or increasing the ionic strength, and/or extracting with a solvent including ionic liquids.

The term "effective," as that term is used in the specification and/or claims, means adequate to accomplish a desired, expected, or intended result.

The use of the word "a" or "an" when used in conjunction with the term "comprising" in the claims and/or the specification may mean "one," but it is also consistent with the meaning of "one or more," "at least one," and "one or more than one."

It is contemplated that any embodiment discussed in this specification can be implemented with respect to any method or composition of the invention, and vice versa. Furthermore, compositions of the invention can be used to achieve methods of the invention.

Throughout this application, the term "about" is used to indicate that a value includes the inherent variation of error for the device, the method being employed to determine the value, or the variation that exists among the study subjects.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprised" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

As used herein, the term "amino" means -NH₂; the term "nitro" means -NO₂; the term "halo" designates -F, -Cl, -Br or -I; the term "mercapto" means -SH; the term "cyano" means -CN; the term "azido" means -N₃; the term "silyl" means -SiH₃, and the term "hydroxy" means -OH.

The term "alkyl" includes straight-chain alkyl, branched-chain alkyl, cycloalkyl (alicyclic), cyclic alkyl, heteroatom-unsubstituted alkyl, heteroatom-substituted alkyl, heteroatom-unsubstituted a)kyl(_{Cn}), and heteroatom-substituted alkyl(_{Cn}). The term "heteroatom-unsubstituted alkyl(_{Cn})" refers to a radical, having a linear or branched, cyclic or acyclic structure, further having no carbon-carbon double or triple bonds, further having a total of n carbon atoms, all of which are nonaromatic, 3 or more hydrogen atoms, and no heteroatoms. For example, a heteroatom-unsubstituted alkyl(_{C1-C10}) has 1 to 10 carbon atoms. The groups, -CH₃ (Me), -CH₂CH₃ (Et), -CH₂CH₂CH₃ (*n*-Pr), -CH(CH₃)₂ (*iso*-Pr), -CH(CH₂)₂ (cyclopropyl), -CH₂CH₂CH₂CH₃ (*n*-Bu), -CH(CH₃)CH₂CH₃ (*sec*-butyl), -CH₂CH(CH₃)₂ (*iso*-butyl), -C(CH₃)₃ (*tert*-butyl), -CH₂C(CH₃)₃ (*neo*-pentyl), cyclobutyl, cyclopentyl, and cyclohexyl, are examples of heteroatom-unsubstituted alkyl groups. The term "heteroatom-substituted alkyl(_{Cn})" refers to a radical, having a single saturated carbon atom as the point of attachment, no carbon-carbon double or triple bonds, further having a linear or branched, cyclic or acyclic structure, further having a total of n carbon atoms, all of which are nonaromatic, 0, 1, or more than one hydrogen atom, at least one heteroatom, wherein each heteroatom is independently selected from the group consisting ofN, O, F, Cl, Br, I, Si, P, and S. For example, a heteroatom-substituted alkyl(_{C1-C10}) has 1 to 10 carbon atoms. The following groups are examples of heteroatom-substituted alkyl groups: trifluoromethyl, -CH₂F, -CH₂Cl, -CH₂Br, -CH₂OH, -CH₂OCH₃, -CH₂OCH₂CF₃, -CH₂OC(O)CH₃, -CH₂NH₂, -CH₂NHCH₃, -CH₂N(CH₃)₂, -CH₂CH₂Cl, -CH₂CH₂OH, CH₂CH₂OC(O)CH₃, -CH₂CH₂NHCO₂C(CH₃)₃, and -CH₂Si(CH₃)₃.

The term "alkenyl" includes straight-chain alkenyl, branched-chain alkenyl, cycloalkenyl, cyclic alkenyl, heteroatom-unsubstituted alkenyl, heteroatom-substituted alkenyl, heteroatom-unsubstituted alkenyl(_{Cn}), and heteroatom-substituted alkenyl(_{Cn}). The term "heteroatom-unsubstituted alkenyl(_{Cn})" refers to a radical, having a linear or branched, cyclic or acyclic structure, further having at least one nonaromatic carbon-carbon double bond, but no carbon-carbon triple bonds, a total of n carbon atoms, three or more hydrogen atoms, and no heteroatoms. For example, a heteroatom-unsubstituted alkeny(_{C2-C10}) has 2 to 10 carbon atoms. Heteroatom-unsubstituted alkenyl groups include: -CH=CH₂ (vinyl), -CH=CHCH₃, -CH=CHCH₂CH₃, -CH₂CH=CH₂ (allyl), -CH₂CH=CHCH₃, and -CH=CH-C₆H₅. The term "heteroatom-substituted alkenyl(_{Cn})" refers to a radical, having a single nonaromatic carbon atom as the point of attachment and at least one nonaromatic carbon-carbon double bond, but no carbon-carbon triple bonds, further having a linear or branched, cyclic or acyclic structure, further having a total of n carbon atoms, 0, 1, or more than one hydrogen atom, and at least one heteroatom, wherein each heteroatom is independently selected from the group consisting of N, O, F, Cl, Br, I, Si, P, and S. For example, a heteroatom-substituted alkenyl(_{C2-C10}) has 2 to 10 carbon atoms. The groups, -CH=CHF, -CH=CHCl and -CH=CHBr, are examples of heteroatom-substituted alkenyl groups.

The term "aryl" includes heteroatom-unsubstituted aryl, heteroatom-substituted aryl, heteroatom-unsubstituted aryl(_{Cn}), heteroatom-substituted aryl(_{Cn}), heteroaryl, heterocyclic aryl groups, carbocyclic aryl groups, biaryl groups, and single-valent radicals derived from polycyclic fused hydrocarbons (PAHs). The term "heteroatom-unsubstituted ary[(_{Cn})" refers to a radical, having a single carbon atom as a point of attachment, wherein the carbon atom is part of an aromatic ring structure containing only carbon atoms, further having a total of n carbon atoms, 5 or more hydrogen atoms, and no heteroatoms. For example, a heteroatom-unsubstituted aryl(_{C6-C10}) has 6 to 10 carbon atoms. Examples of heteroatom-unsubstituted aryl groups include phenyl (Ph), methylphenyl, (dimethyl)phenyl, -C₆H₄CH₂CH₃, -C₆H₄CH₂CH₂CH₃, -C₆H₄CH(CH₃)₂, -C₆H₄CH(CH₂)₂, -C₆H₃(CH₃)CH₂CH₃, -C₆H₄CH=CH₂, -C₆H₄CH=CHCH₃, -C₆H₄C≡CH, -C₆H₄C≡CCH₃, naphthyl, and the radical derived from biphenyl. The term "heteroatom-substituted ary)(_{Cn})" refers to a radical, having either a single aromatic carbon atom or a single aromatic heteroatom as the point of attachment, further having a total of n carbon atoms, at least one hydrogen atom, and at least one heteroatom, further wherein each heteroatom is independently selected from the group consisting of N, O, F, Cl, Br, I, Si, P, and S. For example, a heteroatom-unsubstituted heteroaryl(_{C1-C10}) has 1 to 10 carbon atoms. Examples of heteroatom-substituted aryl groups include the groups: -C₆H₄F, -C₆H₄Cl, -C₆H₄Br, -C₆H₄I, -C₆H₄OH, -C₆H₄OCH₃, -C₆H₄OCH₂CH₃, -C₆H₄OC(O)CH₃, -C₆H₄NH₂, -C₆H₄NHCH₃, -C₆H₄N(CH₃)₂, -C₆H₄CH₂OH, -C₆H₄CH₂OC(O)CH₃, -C₆H₄CH₂NH₂, -C₆H₄CF₃, -C₆H₄CN, -C₆H₄CHO, -C₆H₄CHO, -C₆H₄C(O)CH₃, -C₆H₄C(O)C₆H₅, -C₆H₄CO₂H, -C₆H₄CO₂CH₃, -C₆H₄CONH₂, -C₆H₄CONHCH₃, -C₆H₄CON(CH₃)₂, furanyl, thienyl, pyridyl, pyrrolyl, pyrimidyl, pyrazinyl, quinolyl, indolyl, and imidazoyl.

The term "aralkyl" includes heteroatom-unsubstituted aralkyl, heteroatom-substituted aralkyl, heteroatom-unsubstituted aralkyl(_{Cn}), heteroatom-substituted aralkyl(_{Cn}), heteroaralkyl, and heterocyclic aralkyl groups. The term "heteroatom-unsubstituted aralkyl(_{Cn})" refers to a radical, having a single saturated carbon atom as the point of attachment, further having a total of n carbon atoms, wherein at least 6 of the carbon atoms form an aromatic ring structure containing only carbon atoms, 7 or more hydrogen atoms, and no heteroatoms. For example, a heteroatom-unsubstituted aralkyl(_{C7-C10}) has 7 to 10 carbon atoms. Examples of heteroatom-unsubstituted aralkyls are: phenylmethyl (benzyl, Bn) and phenylethyl. The term "heteroatom-substituted aralkyl(_{Cn})" refers to a radical, having a single saturated carbon atom as the point of attachment, further having a total of n carbon atoms, 0, 1, or more than one hydrogen atom, and at least one heteroatom, wherein at least one of the carbon atoms is incorporated an aromatic ring structures, further wherein each heteroatom is independently selected from the group consisting of N, O, F, Cl, Br, I, Si, P, and S. For example, a heteroatom-substituted heteroaralkyl(_{C2-C10}) has 2 to 10 carbon atoms.

The term "acyl" includes straight-chain acyl, branched-chain acyl, cycloacyl, cyclic acyl, heteroatom-unsubstituted acyl, heteroatom-substituted acyl, heteroatom-unsubstituted acyl(_{Cn}), heteroatom-substituted acyl(_{Cn}), alkylcarbonyl, aryl carbonyl, carbonyl, alkoxycarbonyl and aminocarbonyl groups. The term "heteroatom-unsubstituted acyl(_{Cn})" refers to a radical, having a single carbon atom of a carbonyl group as the point of attachment, further having a linear or branched, cyclic or acyclic, aromatic or non-aromatic structure, further having a total of n carbon atoms, 1 or more hydrogen atoms, a total of one oxygen atom, and no additional heteroatoms. For example, a heteroatom-unsubstituted acyl(_{C1-C10}) has 1 to 10 carbon atoms. The groups, -CHO, -C(O)CH₃, -C(O)CH₂CH₃, -C(O)CH₂CH₂CH₃, -C(O)CH(CH₃)₂, -C(O)CH(CH₂)₂, -C(O)C₆H₅, -C(O)C₆H₄CH₃, -C(O)C₆H₄CH₂CH₃, -COC₆H₃(CH₃)₂, and -C(O)CH₂C₆H₅, are examples of heteroatom-unsubstituted acyl groups. The term "heteroatom-substituted acyl(_{Cn})" refers to a radical, having a single carbon atom as the point of attachment, the carbon atom being part of a carbonyl group, further having a linear or branched, cyclic or acyclic, aromatic or non-aromatic structure, further having a total of n carbon atoms, 0, 1, or more than one hydrogen atom, at least one additional heteroatom, charged or uncharged, in addition to the oxygen of the carbonyl group, wherein each additional heteroatom is independently selected from the group consisting of N, O, F, Cl, Br, I, Si, P, and S. For example, a heteroatom-substituted acyl(_{C1-C10}) has 1 to 10 carbon atoms.. The groups, -C(O)CH₂CF₃, -CO₂H, -CO₂CH₃, -CO₂CH₂CH₃, -CO₂CH₂CH₂CH₃, -CO₃CH(CH₃)₂, -CO₂CH(CH₂)₂, -C(O)NH₂ (carbamoyl), -C(O)NHCH₃, -C(O)NHCH₂CH₃, -CONHCH(CH₃)₂, -CONHCH(CH₂)₂, -CON(CH₃)₂, -CONHCH₂CF₃, -CO-pyridyl, and -CO-pyridiniumyl are examples of heteroatom-substituted acyl groups.

The term "alkoxy" includes straight-chain alkoxy, branched-chain alkoxy, cycloalkoxy, cyclic alkoxy, heteroatom-unsubstituted alkoxy, heteroatom-substituted alkoxy, heteroatom-unsubstituted alkoxy(_{Cn}), and heteroatom-substituted alkoxy(_{Cn}). The term "heteroatom-unsubstituted alkoxy(_{Cn})" refers to a group, having the structure -OR, in which R is a heteroatom-unsubstituted alkyl(_{Cn}), as that term is defined above. Heteroatom-unsubstituted alkoxy groups include: -OCH₃, -OCH₂CH₃, -OCH₂CH₂CH₃, -OCH(CH₃)₂, and -OCH(CH₂)₂. The term "heteroatom-substituted alkoxy(_{Cn})" refers to a group, having the structure -OR, in which R is a heteroatom-substituted alkyl(_{Cn}), as that term is defined above. For example, -OCH₂CF₃ is a heteroatom-substituted alkoxy group.

The term "aryloxy" includes heteroatom-unsubstituted aryloxy, heteroatom-substituted aryloxy, heteroatom-unsubstituted aryloxy(_{Cn}), heteroatom-substituted aryloxy(_{Cn}), heteroaryloxy, and heterocyclic aryloxy groups. The term "heteroatom-unsubstituted aryloxy(_{Cn})" refers to a group, having the structure -OAr, in which Ar is a heteroatom-unsubstituted aryl(_{Cn}), as that term is defined above. An example of a heteroatom-unsubstituted aryloxy group is -OC₆H₅. The term "heteroatom-substituted aryloxy(_{Cn})" refers to a group, having the structure -OAr, in which Ar is a heteroatom-substituted aryl(_{Cn}), as that term is defined above.

The term "aralkyloxy" includes heteroatom-unsubstituted aralkyloxy, heteroatom-substituted aralkyloxy, heteroatom-unsubstituted aralkyloxy(_{Cn}), heteroatom-substituted aralkyloxy(_{Cn}), heteroaralkyloxy, and heterocyclic aralkyloxy groups. The term "heteroatom-unsubstituted aralkyloxy(_{Cn})" refers to a group, having the structure -OAr, in which Ar is a heteroatom-unsubstituted aralkyl(_{Cn}), as that term is defined above. The term "heteroatom-substituted aralkyloxy(_{Cn})" refers to a group, having the structure -OAr, in which Ar is a heteroatom-substituted aralkyl(_{Cn}), as that term is defined above.

The term "acyloxy" includes straight-chain acyloxy, branched-chain acyloxy, cycloacyloxy, cyclic acyloxy, heteroatom-unsubstituted acyloxy, heteroatom-substituted acyloxy, heteroatom-unsubstituted acyloxy(_{Cn}), heteroatom-substituted acyloxy(_{Cn}), alkylcarbonyloxy, arylcarbonyloxy, alkoxycarbonyloxy, aryloxycarbonyloxy, and carboxylate groups. The term "heteroatom-unsubstituted acyloxy(_{Cn})" refers to a group, having the structure -OAc, in which Ac is a heteroatom-unsubstituted acyl(_{Cn}), as that term is defined above. For example, -OC(O)CH₃ is an example of a heteroatom-unsubstituted acyloxy group. The term "heteroatom-substituted acyloxy(_{Cn})" refers to a group, having the structure -OAc, in which Ac is a heteroatom-substituted acyl(_{Cn}), as that term is defined above. For example, -OC(O)OCH₃ and -OC(O)NHCH₃ are examples of heteroatom-unsubstituted acyloxy groups.

The term "alkylamino" includes straight-chain alkylamino, branched-chain alkylamino, cycloalkylamino, cyclic alkylamino, heteroatom-unsubstituted alkylamino, heteroatom-substituted alkylamino, heteroatom-unsubstituted alkylamino(_{Cn}), and heteroatom-substituted alkylamino(_{Cn}). The term "heteroatom-unsubstituted alkylamino(_{Cn})" refers to a radical, having a single nitrogen atom as the point of attachment, further having one or two saturated carbon atoms attached to the nitrogen atom, further having a linear or branched, cyclic or acyclic structure, containing a total of n carbon atoms, all of which are nonaromatic, 4 or more hydrogen atoms, a total of I nitrogen atom, and no additional heteroatoms. For example, a heteroatom-unsubstituted alkylamino(_{C1-C10}) has 1 to 10 carbon atoms. The term "heteroatom-unsubstituted alkylamino(_{Cn})" includes groups, having the structure -NHR, in which R is a heteroatom-unsubstituted alkyl(_{Cn}), as that term is defined above. A heteroatom-unsubstituted alkylamino group would include -NHCH₃, -NHCH₂CH₃, -NHCH₂CH₂CH₃, -NHCH(CH₃)₂, -NHCH(CH₂)₂, -NHCH₂CH₂CH₂CH₃, -NHCH(CH₃)CH₂CH₃, -NHCH₂CH(CH₃)₂, -NHC(CH₃)₃, -N(CH₃)₂, -N(CH₃)CH₂CH₃, -N(CH₂CH₃)₂, *N*-pyrrolidinyl, and *N*-piperidinyl. The term "heteroatom-substituted alkylamino(_{Cn})" refers to a radical, having a single nitrogen atom as the point of attachment, further having one or two saturated carbon atoms attached to the nitrogen atom, no carbon-carbon double or triple bonds, further having a linear or branched, cyclic or acyclic structure, further having a total of n carbon atoms, all of which are nonaromatic, 0, 1, or more than one hydrogen atom, and at least one additional heteroatom, that is, in addition to the nitrogen atom at the point of attachment, wherein each additional heteroatom is independently selected from the group consisting of N, O, F, Cl, Br, I, Si, P, and S. For example, a heteroatom-substituted alkylamino(_{C1-C10}) has I to 10 carbon atoms. The term "heteroatom-substituted alkylamino(_{Cn})" includes groups, having the structure -NHR, in which R is a heteroatom-substituted alkyl(_{Cn}), as that term is defined above.

The term "arylamino" includes heteroatom-unsubstituted arylamino, heteroatom-substituted arylamino, heteroatom-unsubstituted arylamino(_{Cn}), heteroatom-substituted arylamino(_{Cn}), heteroarylamino, heterocyclic arylamino, and alkyl(aryl)amino groups. The term "heteroatom-unsubstituted arylamino(_{Cn})" refers to a radical, having a single nitrogen atom as the point of attachment, further having at least one aromatic ring structure attached to the nitrogen atom, wherein the aromatic ring structure contains only carbon atoms, further having a total of n carbon atoms, 6 or more hydrogen atoms, a total of one nitrogen atom, and no additional heteroatoms. For example, a heteroatom-unsubstituted arylamino(C₆-C₁₀) has 6 to 10 carbon atoms. The term "heteroatom-unsubstituted arylamino(_{Cn})" includes groups, having the structure -NHR, in which R is a heteroatom-unsubstituted aryl(_{Cn}), as that term is defined above. The term "heteroatom-substituted arylamino(_{Cn})" refers to a radical, having a single nitrogen atom as the point of attachment, further having a total of n carbon atoms, at least one hydrogen atom, at least one additional heteroatoms, that is, in addition to the nitrogen atom at the point of attachment, wherein at least one of the carbon atoms is incorporated into one or more aromatic ring structures, further wherein each additional heteroatom is independently selected from the group consisting of N, O, F, Cl, Br, I, Si, P, and S. For example, a heteroatom-substituted arylamino(C₆-C₁₀) has 6 to 10 carbon atoms. The term "heteroatom-substituted arylamino(_{Cn})" includes groups, having the structure -NHR, in which R is a heteroatom-substituted aryl(_{Cn}), as that term is defined above.

The term "aralkylamino" includes heteroatom-unsubstituted aralkylamino, heteroatom-substituted aralkylamino, heteroatom-unsubstituted aralkylamino(_{Cn}), heteroatom-substituted aralkylamino(_{Cn}), heteroaralkylamino, heterocyclic aralkylamino groups, and diaralkylamino groups. The term "heteroatom-unsubstituted aralkylamino(_{Cn})" refers to a radical, having a single nitrogen atom as the point of attachment, further having one or two saturated carbon atoms attached to the nitrogen atom, further having a total of n carbon atoms, wherein at least 6 of the carbon atoms form an aromatic ring structure containing only carbon atoms, 8 or more hydrogen atoms, a total of one nitrogen atom, and no additional heteroatoms. For example, a heteroatom-unsubstituted aralkylamino(_{C7-C10}) has 7 to 10 carbon atoms. The term "heteroatom-unsubstituted aralkylamino(_{Cn})" includes groups, having the structure -NHR, in which R is a heteroatom-unsubstituted aralkyl(_{Cn}), as that term is defined above. The term "heteroatom-substituted aralkylamino(_{Cn})" refers to a radical, having a single nitrogen atom as the point of attachment, further having at least one or two saturated carbon atoms attached to the nitrogen atom, further having a total of n carbon atoms, 0, 1, or more than one hydrogen atom, at least one additional heteroatom, that is, in addition to the nitrogen atom at the point of attachment, wherein at least one of the carbon atom incorporated into an aromatic ring, further wherein each heteroatom is independently selected from the group consisting of N, O, F, Cl, Br, I, Si, P, and S. For example, a heteroatom-substituted aralkylamino(_{C7-C10}) has 7 to 10 carbon atoms. The term "heteroatom-substituted aralkylamino(_{Cn})" includes groups, having the structure -NHR, in which R is a heteroatom-substituted aralkyl(_{Cn}), as that term is defined above.

The term "amido" includes straight-chain amido, branched-chain amido, cycloamido, cyclic amido, heteroatom-unsubstituted amido, heteroatom-substituted amido, heteroatom-unsubstituted amido(_{Cn}), heteroatom-substituted amido(_{Cn}), alkylcarbonylamino, arylcarbonylamino, alkoxycarbonylamino, aryloxycarbonylamino, acylamino, alkylaminocarbonylamino, arylaminocarbonylamino, and ureido groups. The term "heteroatom-unsubstituted amido(_{Cn})" refers to a radical, having a single nitrogen atom as the point of attachment, further having a carbonyl group attached via its carbon atom to the nitrogen atom, further having a linear or branched, cyclic or acyclic structure, further having a total of n carbon atoms, 1 or more hydrogen atoms, a total of one oxygen atom, a total of one nitrogen atom, and no additional heteroatoms. For example, a heteroatom-unsubstituted amido(_{C1-C10}) has 1 to 10 carbon atoms. The term "heteroatom-unsubstituted amido(_{Cn})" includes groups, having the structure -NHR, in which R is a heteroatom-unsubstituted acyl(_{Cn}), as that term is defined above. The group, -NHC(O)CH₃, is an example of a heteroatom-unsubstituted amido group. The term "heteroatom-substituted amido(_{Cn})" refers to a radical, having a single nitrogen atom as the point of attachment, further having a carbonyl group attached via its carbon atom to the nitrogen atom, further having a linear or branched, cyclic or acyclic structure, further having a total of n aromatic or nonaromatic carbon atoms, 0, 1, or more than one hydrogen atom, at least one additional heteroatom in addition to the oxygen of the carbonyl group, wherein each additional heteroatom is independently selected from the group consisting of N, O, F, Cl, Br, I, Si, P, and S. For example, a heteroatom-substituted amido(_{C1-C10}) has 1 to 10 carbon atoms. The term "heteroatom-substituted amido(_{Cn})" includes groups, having the structure -NHR, in which R is a heteroatom-unsubstituted acyl(_{Cn}), as that term is defined above. The group, -NHCO₂CH₃, is an example of a heteroatom-substituted amido group.

Other objects, features and advantages of the present invention will become apparent from the following detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1**: Schematic representation of fluorophore-polymer architectures and mechanisms involved in enzyme mediated photoactivation. Polymeric constructs carrying fluorophore (FL) moieties are non-fluorescent in their native state due to energy transfer; however, upon enzymatic digestion, photoactive-fluorescent fluorophores are released from the conjugate.

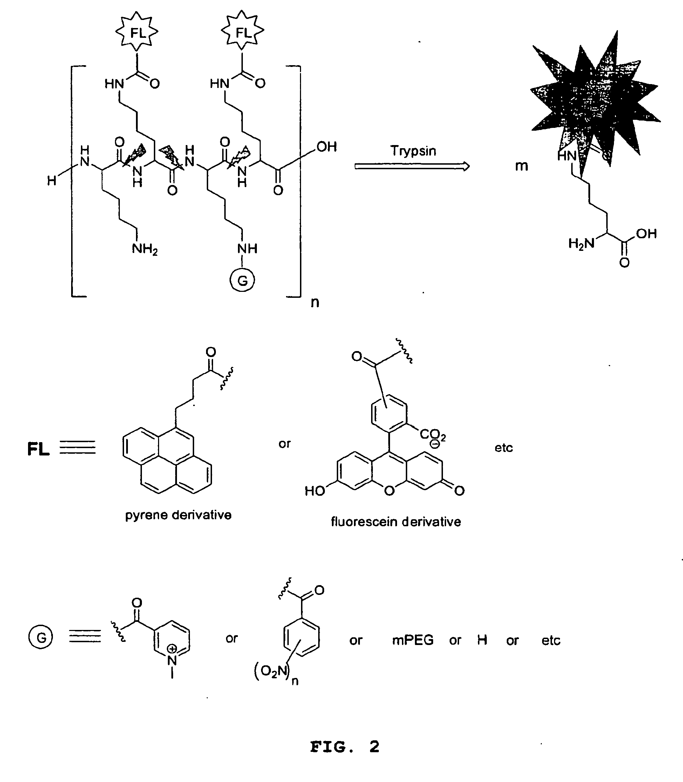

**FIG. 2**: Schematic representation of fluorophore-polymer used to study fluorescence quenching and activation phenomena. The use of amphiphilic architectures, that is, a combination of pyrene as FL and 1-methylnicotinic acid moieties, gave the highest quenching efficiencies. Activation of the conjugates was accomplished by backbone degradation with trypsin.

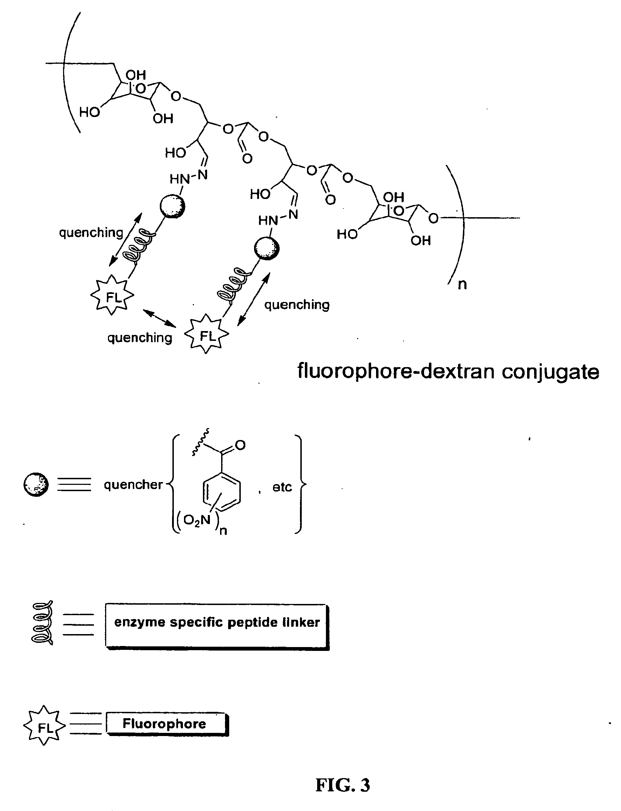

**FIG. 3****:** Schematic representation of a fluorophore-dextran conjugate prepared. Notice that the use of additional quenchers increases energy transfer efficiency.

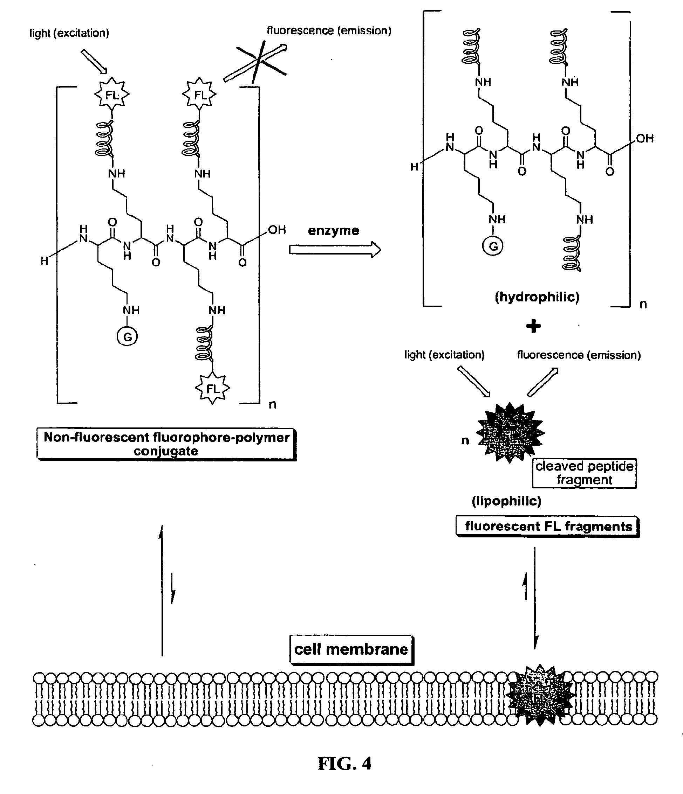

**FIG. 4****:** Schematic representation of the enhanced accumulation of lipophilic fluorophores within targeted tissular compartments after enzymatic cleavage of the conjugate.

### DESCRIPTION OF ILLUSTRATIVE EMBODIMENTS

The present invention overcomes limitations in the prior art by providing imaging probes according to the claims with improved imaging properties; further, the invention provides improved methods for the synthesis of imaging probes. The invention is based, at least partly, on the surprising discovery by the inventor that amphiphilic fluorophore-polymer architectures have advantages over other existing architectures. The imaging probe preferably comprises a plurality of small charged or uncharged moieties to impart one or more desirable characteristics to the imaging probe including: good water solubility, and/or biocompatibility, improved fluorescence or quenching behavior, protection of the polymer backbone from unwanted enzymatic degradation, improved yields of probe preparation, and/or improved probe purification protocols. In particular, the inventors have discovered that the inclusion of a solubilizing moiety (*e.g*., reaction of a polylysine polymer backbone with 1-methylnicotinic acid) can result in a substantial improvement in the characteristics of the imaging probe, such as the quenching of probes in the absence of enzymatic activation.

Methods, kits, and compositions described in the present invention can be used for the selective imaging of cells and tissular structures expressing specific enzymes. For example, in certain embodiments, an imaging agent of the present invention may comprise a solubilizing moiety such as a quaternized ammonium salt, a nitro-phenyl, or a pi-conjugated moiety to improve quenching activation efficiencies. The imaging probes may be used clinically, cosmetically, *in vitro* and *in vivo*, as well as in bacteriology, virology, food technology and agriculture, and for drug screening purposes.

### I. IMAGING PROBES

Imaging probes of the present invention are enzyme-activatible (*i.e.,* cleavage of a bond by an enzyme can increase the fluorescence of the imaging agent) and comprise a polymer backbone conjugated to a plurality (*i.e.,* two or more) of fluorophores. The imaging probe also comprise a plurality of solubilizing moieties and may also comprise one or more of: a quencher, a targeting molecule, and/or a biocompatibilizing units. The inventors have discovered that imaging probes which are amphiphilic macromolecular conjugates possessing a hydrophilic polymer backbone and lipophilic fluorophores which can be used to effectively deliver to a target tissue a load-cargo of lipophilic fluorophores in an inactive form, displaying diminished fluorescence due to energy transfer or self-quenching. However, upon entering the target environment in which certain enzymes are presently active, the conjugate releases its cargo of lipophilic fluorophores due to enzymatic activation of the imaging probe. The fluorophores can then precipitate locally and fluoresce, *e.g.,* upon their diffusion within lipophilic compartments of the target tissue. This creates a highly localized activation of the photoactive agent, which upon light irradiation fluoresces demarcating the target tissue. Also described herein are methods, compositions and kits for the preparation and testing of such fluorophore containing conjugates.

Imaging agents of the present invention, in certain embodiments, can selectively fluoresce in diseased tissues such as cancerous cells or inflamed tissue (*e.g*., breast cancer). Neoplastic tissue possesses several unique characteristics that set it apart form normal tissue, which allows for certain strategies for selective tumor targeting and visualization. The morphological features of most tumors or arthritic tissue reveal "leaky" vasculature due to improper adhesion of vascular endothelial cells, resulting in an enhanced permeation of solutes from the blood stream to the interstitial space of the tumor mass. Secondly, neoplastic cells are known to express specific antigens, which can also be targeted by the use of fluorescently or radio-labeled antibodies. Finally, neoplastic tissue shows clear differences in cellular metabolic activity as compared to normal cells of the same origin. This fact is reflected on several different levels, such as either the overexpression of membrane receptors (folic acid, LDL) or enhanced enzymatic activity, such as proteolytic activity, glucose metabolism, and altered heme biosynthesis. All of the above features can be effectively used for the selective accumulation or uptake of molecular entities for either therapeutic or diagnostic purposes.

Imaging probes of the present invention may, in certain embodiments, have a molecular weight of from about 1kDa to about 100,000 kDa; in various embodiments, the molecular weight of the imaging probe may be from about 15 kDa to about 10,000 kDa, from about 30 kDa to about 5000 kDa, from about 30 kDa to about 1000 kDa, or from about 35 kDa to about 500 kDa.

"Hydrophilic," as used herein, refers to the physical property of a substance to have a preferential affinity for, dissolve in, or physically associate with water. Hydrophilic interactions may involve hydrogen bonding, dipole-dipole, or a charged interaction with water. Various methods for evaluating the hydrophilicity of a compound are known in the art; for example, the log₁₀P of a compound may be measured, wherein P is the partition coefficient (*i.e*., [concentration dissolved in octanol] / [concentration dissolved in water]). According to this test, when P is less than 0, the compound is considered hydrophilic; when P is greater than 0, the compound is considered hydrophobic.

"Hydrophobic" or "lipophilic," as used herein, refers to the physical property of a substance to preferentially associate with or dissolve in organic solvents such as octanol and/or to repel or not associate with water. Various methods for determining the hydrophobicity or lipophilicity of a substance are known in the art. For example the Log₁₀P, wherein P is the partition coefficient, may be evaluated as described above.

As used herein, "functional group" refers to an organic moiety with the potential to either undergo a useful transformation, such as to make a covalent bond, or with the potential to serve a useful purpose, such as impart desired solubility, suppress enzymatic attack, suppress immunological responses. Examples of potentially useful functional groups include but olefins, acetylenes, alcohols, phenols, ethers, oxides, halides, aldehydes, ketones, carboxylic acids, esters, amides, cyanates, isocyanates, thiocyanates; isothiocyanates, amines, hydrazines, hydrazones, hydrazides, diazo, diazonium, nitro, nitrol, mercaptanes, sulfides, disulfides, sulfoxides, sulfones, sulfonic acids, sulfinic acids, acetals, ketals, anhydrides, sulfates, sulfenic acids, amidines, imides, nitrones, hydroxylamines, oximes, hydroxamic acids, thiohydroxamic acids, allenes, ortho esters, sulfites, enamines, amines, ureas, pseudo ureas, semicarbazides, carbodiimides, imines, azides, azo compounds, azoxy compounds, and nitroso compounds.

"Conjugation" or "conjugated" refers to the process of chemically associating or a chemical association between two or more chemical moieties; conjugation may directly associate two moieties, or conjugation may associate two moieties through one or more additional moiety. Conjugation is preferably achieved by the generation of a covalent bond. For example, a polymer backbone may be conjugated to a fluorophore via direct covalent bonding; in other instances, a polymer backbone may be conjugated to a fluorophore indirectly, where the polymer backbone and the fluorophore are both covalently bonded to an enzyme-activatible or enzyme-cleavable linker.

As used herein, "quenching," refers to a process by which part or all of the energy of an excited state of a molecule is altered by a modifying group, such as a quencher. For example, if the excited energy of the modifying group corresponds to a quenching group, then one of the excited triplet states or singlet states of an affected fluorophore is depopulated. It is understood by persons skilled in the art that energy transfer can occur through different mechanisms including FRET or exciplex formation.

"Energy transfer" is well-known to persons skilled in the art, and includes nuclear magnetic energy transfer, transfer of light energy, for example fluorescence energy or phosphorescent energy, Förster transfer, or collisional energy transfer.

### A. Fluorophores

Imaging agents of the present invention comprise a polymer backbone, a plurality of fluorophore moieties and small molecular weight (*e.g*., <lkDa) charged or uncharged protecting/water solubilizing units. The fluorophore moieties are covalently tethered to the polymer either directly or indirectly via an enzyme cleavable linker. In certain embodiments, the fluorophore is a near-infrared fluorophore and/or hydrophobic.

In the absence of enzyme activation, the fluorophore-polymer construct typically has reduced fluorescence due to energy transfer or "self quenching" between fluorophores. This energy transfer is directly proportional to R⁶, where R is the mutual distance between individual fluorophores (Förster, T. "Intermolecular energy transference and fluorescence". Ann Physik 2, 55-75, 1948). In order to improve fluorescence quenching two approaches may be used: 1) increased fluorophore loading onto the polymer and/or 2) increased fluorophore aggregation. The inventor has found that increasing fluorophore loading onto the polymer alone is not always a very practical option for improving quenching due to a gradual decrease in chemical reactivity between the fluorophore component and the polymer as loading increases. Thus, increasing fluorophores interactions (in aqueous-biological media) allows for more effective fluorescence quenching at equivalent fluorophore loadings. Thus, certain imaging probe architectures provided herein do not require high fluorophores loadings, thus making the chemical assembly efficient with high yields.

As used herein, "fluorophore" refers to a molecule or part of a larger molecule, which, when irradiated with light having a wavelength corresponding at least in part to the absorption band(s) of the fluorophore, absorbs part or all of the light energy then emits light at a different (longer or shorter) wavelength in the form of fluorescence.

The fluorophore is hydrophobic and is selected from 1-pyrenebutyric acid (pyrene), NIR-667, NIR-641 N-succinimidyl ester (Fluka), 11-((5-dimethylaminonaphhalene-1-sulfonyl)amino)undecanoic acid (DAUDA), cis-parinaric acid, BODIPY® 581/591, BODIPY® FL (505/511), BODIPY ® 530/550, BODIPY (C1-BODIPY 500/510 C12, C4-BODIPY 500/510 C9, C8-BODIPY 500/510 C5, distyryl-boradiazaindacenes [Z. Dost, S. Atilgan, E. U. Akkaya Tetrahedron 62 (2006), 8484-8488], Dansyl (336/5117), Marina Blue®, M12652 Marina Blue®, pacific blue^{™}, NBD (463/536), fluorescein (496/519), Oregon green® 488 (501/526), tetrametylrhodamine (540/566), lissamine rhodamine (560/581), lissamine rhodamine B sulfonyl chloride, texas rend® (582/601), dansyl undecanoic acid, 6-(N-(7-nitrobenz-2-oxa-1,3-diazol-4-yl)amino)hexanoic acid, 4-fluoro-7-nitrobenz-2-oxa-1,3-diazole, 1-dodecyl,1'-(4-butyric acid NHS)-3,3,3',3'-tetramethylindocarbocyanine halide, N-Ethyl-N-[5-(N-succininmidyloxycarbonyl)pentyl]-3, 3, 3, 3-tetramethyl-2, 2-indocarbocyanine chloride (Dojindo chemicals and Biochemicals), 11-Chloro-1,1'-dipropyl-3,3,3',3'-tetramethyl-10,12-trimethyleneindatricarbocyanine iodide (organica), NIR-664-N-succinimidyl ester (Fluka), 2-[(E)-2-(2-chloro-3-{(E)-2-[3-ethyl-5-phenyl-1,3-benzoxazol-2(3H)-ylidene]ethylidene}-1-cyclopenten-1-yl)ethenyl]-3-ethyl-5-phenyl-1,3-benzoxazol-3-ium iodide (Spectrum), 2-{2-chloro-3-[3-ethyl-1,3-benzothiazol-2(3H)-ylidene]-1-cyclopenten-1-yl}-3-ethyl-1,3-benzothiazol-3-ium perchlorate (Spectrum).

In certain embodiments, a plurality of fluorophores occupy from about 0.1% to about 80%, or from about 3% to about 50% of the available functionalities of said polymer backbone. Similarly, a solubilizing/protecting moiety or other group (*e.g*., see below) may occupy from about 0.1% to about 80%, from about 3% to about 50%, or from about 5% to about 50% of the available functionalities of said polymer backbone. One of skill in possession may routinely modify the degree of substitution of the polymer backbone of an imaging probe as desired (*e.g*., to alter or optimize the physical properties of the imaging probe). For example, fluorophores may occupy about 5%, 10%, 15%, 20%, 25%, 30%, 35%, 40%, 45%, 50%, 55%, 60%, 65%, 70%, 75%, 80%, 85%, 90%, or any range derivable therein, of the available functionalities of a polymer backbone.

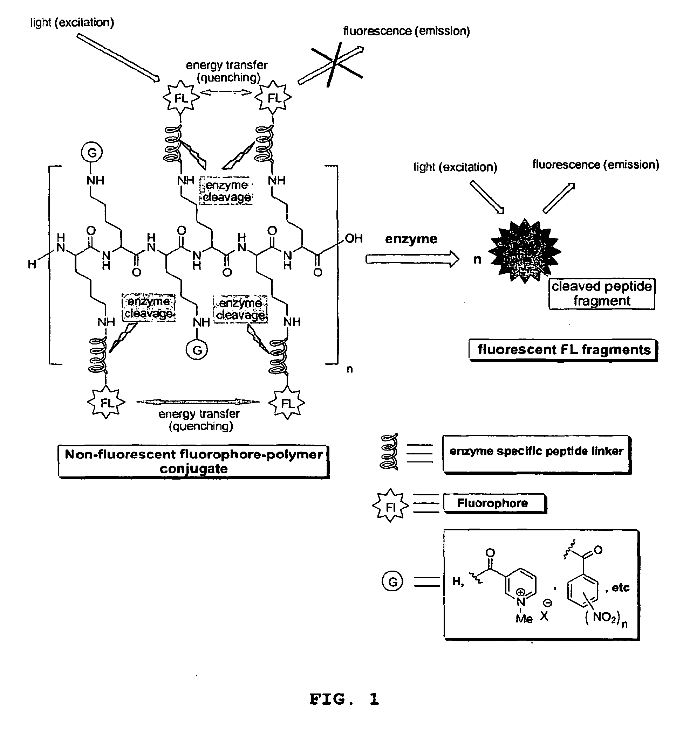

"Available functionalities," as used herein, refers to groups on a polymer which may be used to covalently link another moiety (*e.g*., a fluorophore, an enzyme cleavable linker) to the polymer. For example, poly(L)lysine may be used to form N-epsilon amide bonds with another moiety (see, *e.g*., FIG. 1); if all of the available N-epsilon amide bonds on the poly-L-lysine are covalently linked to, for example, a fluorophore, then 100% of the available functionalities of the polymer are covalently linked to the fluorophore; if, for example, half of the available N-epsilon amide bonds on the poly-L-lysine are covalently attached to fluorophores, then 50% of the available functionalities are bound to fluorophores.

### B. Polymer Backbones

Various polymer backbones are contemplated for inclusion in imaging agents of the present invention. The polymer backbone may be naturally occurring or synthetic. In certain embodiments, the polymer backbone is a polylysine such as poly(L)lysine. Although the backbone is preferably hydrophilic, in certain embodiments a backbone which is essentially neutral may be used.

"Polymer backbone," as used herein, refers to a material made of two or more covalently linked monomer units in a linear or nonlinear fashion. This definition includes dimers, trimers, and higher oligomers, as well as copolymers, block copolymers, and crosslinked polymers. Examples of some useful polymers with the present invention include polylysine, poly-L-lysine, poly-D-lysine, polyarginine, polyornitine, polyglutamic acid, polypeptides comprising of L and/or D amino acids, as well as those comprised of unnatural amino acids, polyvinyl alcohol, polyacrylic acid, polymethacrylate, polyacrylamide, polyalkylcyanoacrylate, polyhydroxyacrylate, polysuccinimide, polysuccinic anhydride, poly(hydroxyethyl methacrylate) (HEMA), HPMA, polysaccharides, polyesters (*e.g*., polylactic acid, polylactides, polyhydroxybutanoates), dextran, aminodextran, modified dextrans, amdex®, oligonucleotides, chitosan, polyurethanes, polycarbonates, polystyrene, polyvinyl alcohol, polyacrylamides, chitosan, trimethyl chitosan, and derivatized chitosan. It also includes polymers that have been modified with additional functionalities in the side chain or the backbone to impart desired physicochemical properties and/or sites for covalent attachment to other molecules such as polystyrene, polystyrene-maleic anhydride, polyesters, polycarbonates, polylactides, polyurethanes, polyethelene, polydivinylbenzene, chitosan-cysteine, chitosan-thioglycolic acid, chitosan-4-thiobutylamidine, polycarbophilcysteamine, and polycarbophil-cysteine. Polymers of the present invention include dendrimers (also called a cascade molecule, a polymer in which the atoms are arranged in multiple branches and/or sub-branches along a central backbone of carbon atoms).

Polymers that may be used with the present invention also include or may comprise a nucleic acid, particularly in embodiments where the enzyme of interest will cleave the backbone sequence in one or more places. As used herein, "nucleic acid" means DNA, RNA, singled-stranded, double-stranded, or more highly aggregated hybridization motifs, and any chemical modification thereof. Modifications include those providing chemical groups that incorporate additional charge, polarizability, hydrogen boding, electrostatic interaction, and fluxionality to the nucleic acid ligand bases or to the nucleic acid ligand as a whole. The nucleic acid may have modified internucleotide linkages to alter, for example, hybridization strength and resistance to specific and non-specific degradation. Modified linkages arc well-known in the art and include methylphosphonates, phosphothioates, phosphodithionates, phospoamidites, and phosphodiester linkages. Alternatively, dephospho-linkages, also well-known in the art, can be introduced as bridges. These include siloxane, carbonate, carboxymethylester, acetamide, carbamate, and thioether bridges.

The term "amino acid" as referred herein, refers to a naturally occurring or synthetic amino acid in a L or D configuration, optionally comprising additional substituents in the alpha position or side chains. Amino acids with unnatural side chains are also specifically contemplated, as are amino acids in which additional methylene units have been introduced into the backbone, such as beta, gamma, delta. In certain embodiments, the amino acid may be a cyclic amino acids in which additional methylene units have been introduced on the backbone or side chains. The use of other amino acid mimics included in this definition are also contemplated and would be recognized by one of skill in the art.

As used herein, "peptide" refers to a polymer of amino acids. Peptides include peptidomimetics, in which either natural or synthetic amino acids are linked by either amide bonds or non-amide bonds (such as peptoids).

### C. Solubilizing Moieties

In various imaging probes of the invention, small molecules are incorporated into a polymer backbone as solubilizing moieties. A solubilizing moiety may be conjugated directly to the polymer backbone or indirectly via an enzyme-cleavable linker. In certain embodiments, the solubilizing moiety has quartenary ammonium species or group.

The solubilizing moieties may be selected from the group consisting of: 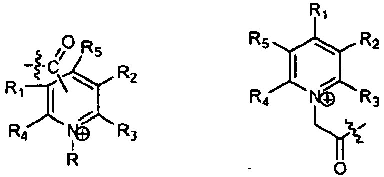 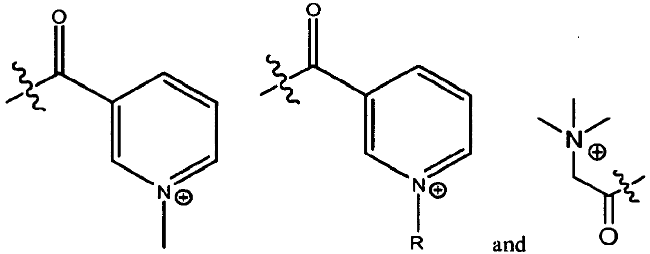 R, R₁, R₂, R₃, R₄, R₅ may each independently selected from the group consisting of H, alkyl(_{C1-6}), hydroxy, amino, cyano, halo, nitro, mercapto, or a heteroatom-substituted or heteroatom-unsubstituted alkyl(_{C1-C10}), aryl(_{C1-C10}), aralkyl(_{C2-C10}), acyl(_{C1-C10}), alkoxy(_{C1-C10}), aryloxy(C₁₋C₁₀), aralkoxy(_{C2-C10}), acyloxy(_{C1-C10}), alkylamino(_{C1-C10}), arylamino(_{C1-C10}), aralkylamino(_{C2-C10}) or amido(_{C1-C10}), or the carbonyl group that conjugates to the hydrophilic polymer backbone as indicated above. The solubilizing moiety may be a nicotinic moiety, and, in certain embodiments, R is -CH₃.

In certain embodiments, one or more of the above solubilizing moieties are produced by chemically reacting a solubilizing compound with an amine group present in a polymer backbone (*e.g*., polylysine) to chemically conjugate the solubilizing moiety to the polymer backbone. Specifically, in certain embodiments, the amide bond may be formed from the reaction between a carboxylic acid residue on a solubilizing compound and an -NH₂ group present on a polymer backbone such as polylysine. For example, the following solubilizing groups may be chemically reacted with a polymer:
Solubilizing compounds which may be reacted with a polymer backbone to form a solubilizing moiety include N-methylnicotinic acid and its derivatives, N-methyl-4-carboxypyridine, N-methyl-2-carboxypyridine, or any other N-alkylcarboxysubstitutedpyridine, N-methylquinoline-X-carboxylic acid (where X=2, 3, 4, 5, or 6, and refers to the position of the carboxyl substituent in the 2, 3, 4, *etc.* position of the pyridine ring), substituted N-methylbenzoquinolines, substituted N-methylacridine, substituted N-methyl isoquinoline, substituted N-methylphenanthredines or any other N-alkylated derivative thereof (*e.g*., where the alkyl is a C₁₋₆ alkyl, a C₁₋₄ alkyl, or a C₁₋₂ alkyl).

For example, N-alkyl substituted-X-carboxypyridine or 1-alkyl substituted-X-carboxypyridine solubilizing moieties, where X=2-6 and refers to the position of the carboxyl substituent (*e.g*., X=3 refers to a nicotinic moiety). Solubilizing compounds specifically envisioned for use with the present invention that may be chemically reacted with a polymer backbone include: 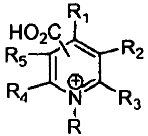 where R=alkyl (*e.g.,* C₁₋₆ alkyl, a C₁₋₄ alkyl, or a C₁₋₂ alkyl) and R₁, R₂, R₃, R₄, R₅, R₆, R₇ and R₈ may be H, alkyl(_{C1-6}), hydroxy, amino, cyano, halo, nitro, mercapto, or a heteroatom-substituted or heteroatom-unsubstituted alkyl(_{C1-C10}), aryl(_{C1-C10}), aralkyl(_{C2-C10}), acyl(_{C1-C2}), alkoxy(_{C1-C10}), aryloxy(_{C1-C10}), aralkoxy(_{C2-C10}), acyloxy(_{C1-C10}), alkylamino(_{C1-C10}), arylamino(_{C1-C10}), aralkylamino(_{C2-C10}) or amido(_{C1-C10}), or the carbonyl group that conjugates to the hydrophilic polymer backbone, provided that one of R₁, R₂, R₃, R₄ and R₅ is said carbonyl group that conjugates to the hydrophilic polymer backbone. In certain embodiments, the alkyl group is a methyl or an ethyl group, as these groups result in reduced lipophilicity.

Other ipso-substituted pyridinium salts such as N or 1-(carboxymethyl)pyridine (shown below) may also be used as solubilizing compounds:

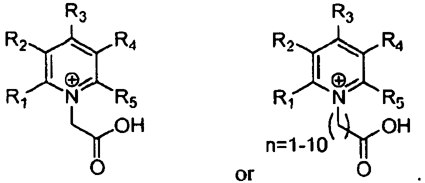

Wherein R, R₁, R₂, R₃, R₄, R₅, R₆, R₇ and R₈ may be H, alkyl(C₁₋₆), hydroxy, amino, cyano, halo, nitro, mercapto, or a heteroatom-substituted or heteroatom-unsubstituted alkyl(_{C1-C10}), aryl(_{C1-C10}), aralkyl(_{C2-C10}), acyl(_{C1-C10}), alkoxy(_{C1-C10}), aryloxy(_{C1-C10}), aralkoxy(_{C2-C10}), acyloxy(_{C1-C10}), alkylamino(_{C1-C10}), arylamino(_{C1-C10}), aralkylamino(_{C2-C10}) or amido(_{C1-C10)}.

As stated above, one or more of the above solubilizing compounds may be chemically reacted with an amine group present in a polymer backbone (*e.g*., polylysine) to chemically conjugate the solubilizing moiety to the polymer backbone via an amide bond. Specifically, in certain embodiments, the amide bond is formed from the reaction between a carboxylic acid residue on the solubilizing moiety and an -NH₂ group present on the polymer backbone.

In certain embodiments, a single type of solubilizing moiety is conjugated to a polymeric backbone. In other embodiments, 2, 3, 4, 5, or more types of solubilizing moieties are conjugated to a polymeric backbone. In various embodiments, a solubilizing moiety of the present invention may be less than about 1000 Da, less than about 900 Da, less than about 800 Da, less than about 700 Da, less than about 600 Da, less than about 500 Da, less than about 400 Da, less than about 300 Da, less than about 200 Da, or less than about 100 Da. The solubilizing moiety or group may be a small molecule group.

Solubilizing moieties do not include polyethyleneglycol or a polyethyleneglycol derivative (*e.g*., mPEG). Nonetheless, polymeric moieties such as polyethyleneglycol or a polyethyleneglycol derivative (*e.g*., mPEG) may be attached to an imaging agent of the present invention. The use of a single polymeric moiety such as polyethyleneglycol, polysaccarides case can result in a block copolymer. In certain embodiments, polyethyleneglycol or a polyethyleneglycol derivative is used in combination with a solubilizing moiety according to the claims such as 1-methylnicotinic acid.

Without limiting the invention to any single theory, the surprising discovery that such simple nitro-phenyl moieties increase the quenching efficiency of fluorophore-polymer probes might be due to variety of factors such as either increased fluorophore-fluorophore interactions through pi donor acceptor or other interactions. These relatively simple and inexpensive moieties thus provide substantial advantages as additional fluorescence quenchers/solubilizing moieties. The inventor has further discovered that the inclusion of these quenchers at the opposite end of the fluorophore on the cleavable peptide sequence gave probes with excellent quenching/activation properties even with very low fluorophore loadings.

Solubilizing moieties conjugated to imaging probes of the present invention are hydrophilic. In certain embodiments the solubility (mM in water) of the solubilizing moiety or solubilizing compound is greater than 0.3, more preferably greater than 0.4, more preferably greater than 0.5, more preferably greater than 0.6, more preferably greater than 0.7, more preferably greater than 0.8, more preferably greater than 0.9, more preferably greater than 1, more preferably greater than 2, more preferably greater than 3, more preferably greater than 4, more preferably greater than 5, more preferably greater than 6, more preferably greater than 7, more preferably greater than 8, more preferably greater than 9, and most preferably greater than 10.

### D. Protecting Groups

In certain embodiments, an imaging agent may comprise one or more protecting groups. A "protecting group," "protecting moiety," and "protecting units," as used herein, refers to a small chemical entity of natural or synthetic origin that serves to shield the polymeric backbone from enzymatic degradation by masking key enzymatic recognition sites of the substrate. In certain instances a single moiety may serve as both a protecting moiety and a solubility moiety. In certain embodiments, a protecting group may be attached to a polymer backbone (*e.g*., poly(L)lysine) or to an enzyme-cleavable linker (*e.g*., cleavable by cathespin D) via an amide bond (*e.g*., via NHS ester coupling of the protecting moiety to, for example, available epsilon-amine groups of lysine comprised in a poly-lysine or a poly(L)lysine polymer backbone).

These protecting groups include amide, imide, imine, ester, thioester, carbazone, hydrazones, oxime, acetal, and ketal, phosphatidyl choline and its derivatives, N-methylnicotinic acid and its derivatives, N-methyl-4-carboxypyridine, N-methyl-2-carboxypyridine, or any other N-alkylcarboxysubstitutedpyridine, N-methylquinoline-X-carboxylic acid (where X=2, 3, 4, 5, 6, or 7), substituted N-methylbenzoquinolines, substituted N-methylacridine, substituted N-methyl isoquinoline, substituted N-methylphenanthredines or any other N-alkylated derivative thereof. It also includes substituted N-alkylated pyridine containing systems, such as substituted pyridines, benzopyridines, dibenzopyridines. The mentioned substituents include carboxylic acid and esters, aldehydes, ketones, amine, alcohols. These "protecting units" also include monovalent derivatization with dicarboxylic acids, including oxalic, maleic, succinic, glutaric, adipic acid, or monovalent derivatization with polycarboxylic acids, including citric acid, or natural or unnatural amino acids or peptides, in which the amine functions may or may not be quaternized by methyl or any other alkyl group. Alkylation of amines can also be used to quaternized polymeric amine functions. Other protecting functionalizations include derivatization with sulfoacids, such as sulfoacetic acid, ascorbic acid-2-sulfate, or O-sulfonated amino acids, such as O-sulfo-serine, O-sulfo-tyrosine, O-sulfo-threonine or O-sulfonated saccarides or polysaccarides or peptides. Similarly, derivatization with phosphorylated acids and amino acids, such as phosphogliceric acid, O-phospho-serine, O-phospho-threonine, O-phospho-tyrosine, ascorbic acid-2-phosphate (vitamin C phosphate), or O-phosphorylated saccarides or polysaccarides such as glyceraldehyde-3-phosphate, glucose-6-phosphate, erythrose-4-phosphate, ribose-5-phosphate, pyridoxal-5-phosphate, glusosaminc-6-sulfate. Derivatization with saccarides and disaccarides including glucose, galactose, fructose, sucrose, mannose, maltose, isomaltose, cellobiose, L-iduronic acid.

### E. Enzyme-Cleavable Linkers

In certain embodiments, an imaging probe may comprise an enzyme-cleavable linker. For example a fluorophore or quencher moiety may be conjugated to a polymer backbone via an enzyme-cleavable linker; in these instances, cleavage of the enzyme-cleavable linker results in the release of the fluorophore or quencher moiety from the polymer backbone. In other instances it may be desirable to have one or more enzyme-cleavable linkers on the polymer backbone itself; thus, in these instances, cleavage of the polymer backbone may result in a similar increase in the fluorescence of such imaging agents.

"Enzyme-cleavable linker" or "enzyme cleavable linker" as used herein, refers to a monomer or polymer unit which can be cleaved by an enzyme and can be used to covalently bind a polymer and an additional moiety or moieties, such as a chromophore, fluorophore, quenchers, blackhole quenchers, gold nanoparticles, quantum dots, or iron oxide nanoparticles. The enzyme-cleavable linker may be a natural or unnatural amino acid, a peptide made of natural L and/or D amino acids, a peptide made of unnatural amino acids, a saccaride, a polysaccharide, an oligonucleotide, an oligonucleotide with modified nucleobases and/or modified backbone, a natural or synthetic molecule which serves as an enzymatic substrate. As stated below, several enzyme-cleavable linkers are known and may be used with the present invention.

### F. Quencher Moieties

Imaging probes of the present invention may, in certain embodiments, comprise a quencher moiety. Quencher moieties reduce the fluorescence of a fluorophore via interacting with the fluorophore. Quenchers are known in the art and include additional fluorescent or non-fluorescent fluorophores, fluorescent or non-fluorescent quenchers, nitro-substituted phenyl moieties, black hole quenchers, quantum dots, gold nanoparticles. In various embodiments, a quencher moiety may be conjugated to a polymer backbone either directly or via an enzyme-cleavable linker. Certain quenching moieties may function as biocompatibilizing unit and/or protecting moiety.

Quenchers also include p-nitrosubstituted phenyl moieties such as p-nitrobenzoic acid, m-nitrobenzoic acid, o-nitrobenzoic acid, dinitrobenzoic acid, trinitrobenzoic acid, dinitrophenol, picricsulfonic acid, nitrophenylsufonyl chlorides.

Without limiting the invention to any theory, the surprising discovery that such simple nitro-phenyl moieties increase the quenching efficiency of fluorophore-polymer probes might be due to variety of factors such as either increased fluorophore-fluorophore interactions through pi donor acceptor or other interactions. These relatively simple and inexpensive moieties thus provide substantial advantages as additional fluorescence quenchers/solubilizing/protecting moieties. The inventor has further discovered that the inclusion of these quenchers at the opposite end of the fluorophore on the cleavable peptide sequence gave probes with excellent quenching/activation properties even with very low fluorophore loadings.

Multiple quenchers are known in the art and may be conjugated to a polymer backbone. Quenchers specifically contemplated for use with the present invention include:
a) non-fluorescing dyes such as DABCYL; DANSYL;QSY-7, Black Hole Quenchers;
b) fluorophores, including commercially available fluorescent labels from the SIGMA chemical company (Saint Louis, MO), Molecular Probes (Eugene, OR), R & D systems (Minneapolis, MN), Pharmacia LKB Biotechnology (Piscataway, NJ), CLONTECH Laboratories, Inc. (Palo Alto, CA), Chem Genes Corp., Aldrich Chemical Company (Milwaukee, WI), Glen. Inc., GIBCO BRL Life Technologies, Inc. (Gaithersburg, MD), Fluka Chemica-Biochemika Analytika (Fluka Chemie AG, Buchs, Switzerland), and Applied Biosystems (Foster City, CA), as well as many other commercial sources known to one of skill;
c) moieties derived from nitro-substituted phenyl compounds including *p*-nitrobenzoic acid, *m*-nitrobenzoic acid, *o*-nitrobenzoic acid, 3,5-dinitrobenzoic acid, 2,4,6-trinitrobenzenesulfonic acid,
d) Nano-scaled semiconductors, such as quantum dots, nanotubes, and other quantum-well structures.

### G. Biocompatibilizing Units

Imaging probes of the present invention may comprise a biocompatibilizing unit. As used herein, a "biocompatibilizing unit" or "biocompatibility moiety" refers to any natural or synthetic moiety may be conjugated to an enzyme-activable fluorophore in order to alter its pharmacokinetic profile, modify its biodistribution or clearance, and/or to protect the polymeric backbone from unwanted degradation. Examples for such entities are well known in the art and include negatively charged units such as, *e.g*., succinic acid, sulfopropionic acid, and/or negatively charged units, including N-methylnicotinic acid, N-methyl-4-carboxypyridine, N-methyl-2-carboxypyridine, or any other N-alkylcarboxy substituted pyridine, saccarides. Alternatively, the use of a single polymeric moiety comprised of polyethylene glycol, polyethylene glycol copolymers, dextrans, cyclodextran, polysaccarides will result in block copolymers. Certain moieties may function as a biocompatibilizing unit and/or a solubility moiety and/or a quencher.

### H. Targeting Moieties

In certain embodiments, the imaging probe may comprise a targeting moiety. The inclusion of a targeting moiety in an imaging probe may be used to improve the intracellular bioavailability of an imaging probe for more effective *in vivo* and *in vitro* fluorescence imaging. The ligand binding targeting moiety (targeting moiety) includes cell surface recognizing molecules and molecules with a specific affinity for a cell surface component (e.g., which is specific for the cell type/tissue of interest, such as a cancerous cell/tumor). In certain embodiments, the cell surface component enhances uptake of the imaging agent into a cell or tissue of interest.

Targeting moieties can improve the selectivity of the imaging probes towards a specific tissue or pathology; for example, a targeting moiety may enhance uptake and/or accumulation in specific tissues (*e.g*., at cells of a specific tissuc, or at diseased cells such as cancerous cells). Targeting moieties include peptides, peptides with L and/or D configured amino acids, peptides with unnatural amino acids, folic acid, cholesterol esters, cell adhesion molecules (RGD peptides), steroids, modified steroids, saccharides, polysaccharides, oligonucleotides, folic acid, cholesterol, cholesterol esters, antibodies. The attachment between the polymer and the targeting moiety is preferably a covalent bond but in some instances may also be a non-covalent bond.

As used herein, "target" refers to any molecule, enzyme, receptor, cell membrane, protein, antibody, antigen, tissue, or pH of interest. A specific target is chosen to impart greater selectivity to the conjugate by improving its affinity towards pathological regions. For instance, neoplastic cells can be selectively targeted by exploiting over-expression of cell adhesion receptors (RGD), folic acid receptors, LDL receptors, insulin receptors and glucose receptors; in addition, neoplastic cells are known to express cancer specific antigens. A target can also be, for example, an enzyme (metallomatrix proteases, cathepsin), nucleic acid, peptide, protein, polysaccharide, carbohydrate, glycoprotein, hormone, receptor, antibody, virus, substrate, metabolite, cytokine, inhibitor, dye, growth factor, nucleic acid sequence, pH value.

### II. ENZYMATIC ACTIVATION OF IMAGING PROBES (SPECIFIC ENZYMES AND SEQUENCES)

Enzyme-mediated fluorescence activation of the probe requires substrate access by the enzyme of interest. For example, probes made by directly tethering the fluorophore to the polymer backbone, fluorescence restoration involves access and cleavage of the polymer backbone by the enzyme. For the other case, the enzyme must access and cleave the linkers joining the polymer and the fluorophore.

The enzyme-activatable increase in fluorescence is caused by cleavage of a bond in the imaging probe by an enzyme such as, for example, trypsin, a cathespin, cathespin D, cathespin B, cathespin H, cathepsin L, cathepsin S, urokinase, thrombin, plasmin, a plasminogen activator, a prostate specific antigen, a matrix metalloproteinase (MMP), a kallikrein, a human kallikrein, or human kallikrein 3, a caspase, a caspase 3, a caspase 8, a granzyme B, a calpain. Specific enzyme cleavable linkers (*e.g*., moieties and/or amino acid sequences) that may be cleaved by these enzymes are known in the art (*e.g*., Asp-Val-Glu-Asp- to target caspase 3 [Bioorg Med Chem Lett 1999, 9, 3231-3236 J. Liu, M. Bhalthat, C. Zhang, Z. Diwu], Ile-Glu-Thr-Asp- to target caspase 8, Ala-Ala-Asp- to target granzyme B [J Biol Chem 2001, 276, 6974] Leu-Met- to target calpain [J Biol Chem 1993, 268, 23593 B. G. Rosser, S. P. Powers, G. J. Gores],) and are specifically contemplated for inclusion in imaging probes of the present invention. In certain embodiments, an imaging probe comprises a cathespin D cleavable linker (*e.g.*, Gly-Pro-Ile-Cys(Et)-Phe-Phe-Arg-Leu-Gly). In further embodiments, a fluorophore is conjugated to a polymer backbone (*e.g.*, poly(L)lysine) via an enzyme-cleavable linker.

The table below provides additional examples of possible enzyme-cleavable linkers that may be included in imaging probes according to the present invention.

**TABLE 1**

| Sequence | Target | References: |
|---|---|---|
| Pro-Leu-Gly/Leu-Trp-Ala-D-Arg-NH2 | MMP-1 | Stack M.S. et al. J.Biol.Chem. 264, 4277-4281 |
| Pro-Cha-Abu/Smc-His-Ala-D-Arg-NH2 (Cha : cyclohexylalnine, Abu: alpha-aminobutyric acid Smc: S-methylcysteine) | MMP-1 | McGeehan G.M.et al. J.Biol.Chem. 269, 32814-32820) |
| Lys-Pro-Leu-Ala/Nva-Asp-Ala-Arg-NH2 | MMP-2 | Anaspect brochure |
| *Pro-Leu-Gly/Leu-Ala-Arg-NH2 | MMP-2 | (Knight C.G., FEBS 296, 3, 263-266) |
| Arg-Pro-Lys-Pro-Tyr-Ala/Nva-Trp-Met-Lys-NH2 | MMP-3 | Nagase H. et al. J. of Biol. Chemistry 1994, 269(33) 20952-20957 |
| Arg-Pro-Lys-Pro-Val-Glu/Nva-Trp-Arg-Lys-NH2 | MMP-3 | Nagase H. et al. J. of Biol. Chemistry 1994, 269(33) 20952-20957 |
| Arg-Pro-Leu-Ala/Leu-Trp-Arg-Ser(AHX)Cys (where AHX stands for aminohexanoic acid) | MMP-7 | McIntyre JO, Biochem J. 2004 Feb 1;377(Pt 3):617-28. |
| Arg-Pro-Leu-Ala/Leu-Trp-Arg-Ser | MMP-7 | Welch A.R. et al. 1995, Arch. Biochem. Biophys. 324. 59 |
| Pro-Leu-Gly-Leu-Dpa-Ala-Arg-NH2 | MMP-7 | (Knight C.G., FEBS 296. 3, 263-266) |
| Ser-Gly-Lys-Gly-Pro-Arg-Gln/Ile-Thr-Ala | MMP-9 | Kridel S.J. J. of Biol Chem 2001, Vol 276, 23(8), 20572-20578 |
| Ser-Gly-Lys-Ile-Pro-Arg-Arg/Leu-Thr-Ala | MMP-9 | Kridel S.J, J. of Biol Chem 2001. Vol 276, 23(8), 20572-20578 |
| Pro-Gln-Gly-Ile-Ala | MMP-9 | Williams H.R et al. Int. J. Biochem. 1984 (16) 1321-1329 |
| *Pro-Cha-Gly/Nva-His-Ala-NH2 | MMP-13 | Knäuper V. et al., J. of Biol. Chem. 1996. Vol 271. (3) 1544-1550 |
| *Pro-Leu-Gly/Leu-Ala-Arg-NH2 | MMP-13 | Knäuper V. et al., J. of Biol. Chem. 1996. Vol 271, (3) 1544-1550 |
| Gly-Pro-Leu-Gly-Meth-Arg-Gly-Leu-NH2 | MMP-13 | Deng S.J et al. J. of Biol. Chem. 2000, Vol. 275, No 40, 31422-31427 |
| pGSGR/SAG PGSGR/SASGTTGTG | uPA | Coombs G. S et al (1998) |
| SGR/SA GSGK/S | uPA | Ke S. H el al. (1997) |
| D-Phe-Pip-Arg-pNA D-Phe-Pro-Arg-pNA | Thrombin | Rijkers D. T. S. et al. (1995) |
| Ac-Nle-Thr-Pro-Arg-AMC Ac-Val-Thr-Pro-Arg-AMC | Thrombin | Backes B J et al. (2000) Harris J L et al. (2000) |
| Gly-Ser-Pro-Ala-Phe-Leu-Ala-Lys-D-Arg- | *Cathepsin E* | |
| Lys-Pro-Leu-Gly-Leu-Dap-Ala-Arg | *MMPs. Cathepsin D and E. ADAM10 and ADAM17*/*TACE* | |
| Lys-Pro-Leu-Gly-Leu-A2pr-Ala-Arg- | *Matrix Metalloproteinases, and ADAM17*/ *Tumor Necrosis Factor Converting Enzyme (TACE)* | |
| Pro-Cha-Gly-Nva-His-Ala-Dap | *MMPs* | |
| Ser-Glu-Val-Asn-Leu-Asp-Ala-Glu-Phe-Arg-Lys-Arg-Arg | β*-Cleavage Site of Swedish-Type Amyloid Precursor Protein* | |
| Gly-Gly-Val-Val-Ile-Ala-Thr-Val-Lys-D-Arg-D-Arg-D-Arg | *gamma-secretase* | |
| Gly-Lys-Pro-Ile-Leu-Phe-Phe-Arg-Leu-Lys-D-Arg | *Cathepsin D and E* | |
| Asp-Glu-Val-Asp-Ala-Pro-Lys- | *Caspase-3* | |
| Ala-Pro-Ala-Lys-Phe-Phe-Arg-Leu-Lys | *Proteinase A* / *Pepsin* | |
| Tyr-Val-Ala-Asp-Ala-Pro-Lys- | *Caspase-1* | |
| Pro-Cha-Gly-Cys(Me)-His-Ala-Lys | *Collagenase* | |
| Leu-Ala-Gln-Ala-Val-Arg-Ser-Ser-Ser-Arg-Dap- | *ADAM17*/*TNF*-α *Converting Enzyme* | |
| Gly-Phe-Ser-Pro-Tyr(NO2) | *ACE2* | |

| | | |
|---|---|---|
| * MCA as fluorophore and N-3-(2,4-dinitrophenyl)-L-2,3-diaminopropionyl (Dpa) as quencher moiety Nva: L-norvaline | | |

Without being bound by any theory, increases in enzymatic access to clevage sites may contribute to the advantages of using a small hydrophilic molecule as solubilizing agent as compared to other moieties (*e.g.*, PEG). The inventor has observed that probe digestion/activation by enzymes becomes slower by increasing the percentage of loading of fluorophore on the polymer. It was also found that PEGylation of the polymer backbone dramatically decreased the rate of probe enzymatic activation. In addition, PEGylation also decreased fluorescence quenching and thus required higher fluorophore loadings to achieve sufficient fluorescence quenching. On the other hand, the use of small charged or small hydrophilic moieties allowed producing probes with much better enzymatic activation characteristics for the following main reasons: I) the intrinsic fluorescence of the undigested probe is very low, allowing for better contrast, 2) well quenched probes did not required very high fluorophore loadings and/or 3) the small moieties did not induce steric hindering of substrate sites by the enzyme, and/or 4) efficient chemical assemblage of the imaging probe.

### III. METHODS FOR MAKING IMAGING PROBES

Tethering of units to the polymer backbone is preferably accomplished through covalent bonds which are preferably made under mild reaction conditions. Preferred classes of reactions for conjugating one or more moiety to a polymer backbone include those which proceed under relatively mild conditions. These include nucleophilic substitutions (*e.g.*, reactions of amines, hydrazines, hydrazides, thiols and alcohols with acyl halides, active esters, carbonyls, and carbon-halide bonds), electrophilic substitutions (*e.g.*, enamine reactions) and additions to carbon-carbon and carbonheteroatom multiple bonds (*e.g.*, Michael reaction, Diels-Alder addition).

Several approaches are available for conjugating an enzyme cleavable linker to a polymer backbone and/or a fluorophore. In certain embodiments, specific peptide sequences may be used as enzyme cleavable linkers for this purpose, and peptide sequences possess the advantage of being easily assembled on the solid-phase by the Fmoc or Boc strategy. The fluorophore units can easily be installed on the peptide via terminal or side chain NH₂ functions (using activated esters of a fluorophore, Michael additions), as well as OH, SH, and carboxylic functions. In certain embodiments, terminal or lysine side chains NH₂ functions or ornitine NH₂ functions of a peptide polymer backbone or peptide (enzyme-cleavable linker) are preferably reacted in combination with an activated ester of a fluorophore.

It is also possible to use modified or unnatural amino acids to conjugate a peptide (*e.g.*, a polymer backbone or an enzyme-cleavable linker) to a fluorophore involving various functional groups and chemical reactions (*e.g.*, Koehn and Breinbauer, 2004; Breinbauer and Koehn, 2003; Kolb *et al.,* 2003; Veronese *et al.,* 1999; Means and Feeney, 1998; Mattoussi et *al.,* 2004; Hoffman and Stayton, 2004). It is also possible to tether the fluorophore to the peptide on the solid-phase. This procedure offers the possibilily of carrying out the coupling reaction of the fluorophore chemoselectively on a fully or partially protected peptide; subsequent release from the solid phase can then yield the enzyme-cleavable linker with the fluorophore already installed (*e.g.*, see Examples). Nevertheless, it is also possible to first obtain a fully deprotected peptide and carry out the coupling to the fluorophore chemoselectively in solution (Licha *et al.*, 2002; Rau *et al.*, 2001; Ching-Hsuan *et al.*, 1999). Similarly, other enzyme cleavable linkers can be employed including saccharides, polysaccharides, polyesters, and oligonucleotides to target a known over expressed enzyme which is associated with a targeted pathology.

Oligonucleotides linkers (serving as enzyme-cleavable linkers) may be synthesized by a number of different approaches including commonly known methods of solid-phase chemistry. Linkers bearing a photosensitizer in one end and a spacer with the appropriate functional group at the opposite end can be synthesized on an automated DNA synthesizer (*e.g.* P.E. Biosystems Inc. (Foster Clif, CA) model 392 or 394) or using standard chemistry, such as phosphoramidite chemistry (Ozaki and McLaughlin, 1992; Tang and Agrawal, 1990; Agrawal and Zamecnik, 1990; Beaucage, 1993; Boal *et al.*, 1996). When using automated DNA synthesizers, the fluorophore and spacers have the advantage of being introduced during automated synthesis. Alternatively, one or more of these moieties can be introduced either before or after automated synthesis. Additional strategies for conjugation to growing or complete sequences will be apparent to those skilled in the art. Following automated synthesis the reaction products may be cleaved from their support, protecting groups removed and the linker-fluorophore be purified by methods known in the art, *e.g.* chromatography, extraction, gel filtration, or high pressure liquid chromatography (HPLC).

The enzyme-cleavable linker can be tethered to the conjugate chemoselectively. For this purpose, a chemoselective functional group pair must be utilized. Chemoselective functional groups include amine-activated ester, hydrazine derivatives-activated ester, hydrazine derivatives-carbonyl, thiols-substitution reactions (carbon-halide bonds, alkylsulphonic esters), thiols-Michael additions (maleiimides, acrylates, vinylsulphones, vinylketones) thiols-thioligation or natural chemical ligation (requires either an N-terminal cysteine with a thioester, or 1-hydroxy-8-sulfenyl dibenzofuran moiety with a thiol, or aminoethane sulphonyl azides with thio acids), thiol-disulfide bonds, amines-substitution reactions (activated carbon-halide bonds, activated esters, and activated alkylsulphonic esters), amines-Michael additions (maleiimides, acrylates, vinylsulphones, vinylketones), diels-alder reactions (requiring a diene and a dienophile), 1,3-dipolar additions. The proper choice of chemoselective reaction will be obvious to one skilled in the art (For reviews see, *e.g.*, Koehn and Breinbauer, 2004; Breinbauer and Koehn, 2003; Kolb *et al.,* 2003; Veronese and Morpurgo, 1999; Means and Feeney, 1998; Mattoussi *et al.*, 2004; Hoffman and Stayton, 2004).

Certain imaging probes of the present invention may be synthesized as follows: A predefined amount of the solvent in a container (*e.g.*, labeled "a") is transferred into a vial (*e.g.*, labeled "b") containing the base. The mixture is shaken or mixed until homogeneous and a defined amount of this basic solution transferred into a vial (labeled "c") which contains the peptide with deprotected N- and C-termini and the mixture shaken or mixed until a homogenous solution is obtained. All of the contents from vial (labeled c) are then transferred to a vial (*e.g.*, labeled "d") which contains the activated fluorophore and the mixture shaken or mixed until a homogenous solution is obtained. The coupling reaction between the peptide N-terminus and the fluorophore is then typically allowed to proceed for about 4-8 hours. Thereafter, another defined amount of the solvent in container (labeled a) is transferred into a container (*e.g.*, labeled "e") containing the polymer, and the mixture shaken or mixed until a homogeneous solution is obtained. The contents of this vial containing the dissolved polymer (labeled e) are then transferred into the container (labeled d) containing the fluorophore-peptide conjugate and the mixture shaken or mixed until a homogeneous solution is obtained. Finally, the contents of this vial (labeled d) containing the polymer, the base and the fluorophore-peptide conjugate are added to a vial (*e.g.*, labeled "f"), which contains the activating/coupling reagent. The mixture is shaken until a homogeneous solution is obtained and coupling reaction between polymer and fluorophore-peptide conjugate allowed to proceed for 8 h. At this point, all of the solution containing the fluorophore-polymer conjugate is transferred to vial labeled g) and the mixture shaken until a homogeneous solution is obtained. Reaction is allowed to proceed for 2 h then excess solvent and base is removed by repeated extractions with diethyl ether until the reaction volume reaches a certain value. The product is finally purified by either passing through a desalting column or SEC column or ion exchanged resin column using water as eluent. The final product is can be used as obtained or lyophilized for storage.

In certain embodiments, imaging a tissue or cells expressing a target enzyme may comprise the following steps:
a) topical or systemic administration of an adequate amount of said enzyme-activatable fluorophore-polymer conjugate in a pharmaceutically acceptable composition according to this invention to an *in vivo* or *in vitro* subject
b) permitting sufficient time to elapse, allowing for the uptake, accumulation and activation of an effective amount of fluorophore-polymer conjugate according to this invention in the target
c) irradiation of a target area of the subject with light having a wavelength corresponding at least in part to the absorption bands of the enzymatically cleaved fluorophores.
d) monitoring the fluorophores fluorescence using an adequate imaging device.

Various methods may be used to conjugate a functional group to a polymer backbone. For example, these functional groups include:
a) carboxyl groups and various derivatives thereof such as N-hydroxysuccinimide esters, N-hydroxybenzotriazole esters, acid halides, acyl imidazoles, thioesters, p-nitrophenyl esters, alkyl, alkenyl, alkynyl and aromatic esters;
b) hydroxyl groups, which can be converted to esters, ethers, aldehydes,
c) haloalkyl groups, wherein the halide can be later displaced with a nucleophilic group such as, for example, an amine, a carboxylate anion, thiol anion, carbanion, or an alkoxide ion, thereby resulting in the covalent attachment of a new group at the site of the halogen atom;
d) dienophile groups, which are capable of participating in Diels-Alder reactions such as, for example, maleimido groups;
e) aldehyde or ketone groups, such that subsequent derivatization is possible via formation of carbonyl derivatives such as, for example, imines, hydrazones, semicarbazones or oximes, or via such mechanisms as Grignard addition or alkyllithium addition; sulfonyl halide groups for subsequent reaction with amines, for example, to form sulfonamides;
g) thiol groups, which can be, for example, converted to disutfides or reacted with acyl halides;
h) amine or sulfhydryl groups, which can be, for example, acylated, alkylated or oxidized;
i) alkenes, which can undergo, for example, cycloadditions, acylation, Michael addition;
j) epoxides, which can react with, for example, amines and hydroxyl compounds; and thiols.
(k) phosphoramidites and other standard functional groups useful in nucleic acid synthesis.

### A. Kits

The present invention specifically contemplates kits comprising an imaging agent of the present invention; the kit may be used for imaging purposes *in vivo* and *in vitro.* Kits may contain one or more fluorophore-polymer conjugates and instructions for their preparation. Optionally, kits according to this invention may include enzymes, reagents and other devices so that the user of the kit may easily use it for the preparation of fluorophore-polymer conjugates directed against a preselected enzyme target.

In various embodiments, the kit may comprise 1, 2, 3, 4, 5, 6, 7 or all of the following:
a) first container means containing a solvent, such as DMSO or DMF or water a combination of these solvents or other suitable solvents or combinations thereof;
b) a second container means containing a specific amount of the base, such as diisopropylethylamine, triethyl amine, pyridine, or any other suitable base including those that are solid supported;
c) a third empty container means in which one may place a predetermined amount of the a synthesized (*e.g.*, in-house synthesized), amine-reactive, N- and C deprotected peptide (linker) with side chains either fully protected, partially protected, or not protected.
d) a fourth container means containing an activated fluorophore for suitable coupling via the amine reactive functionality of the peptide (linker) such as either an active ester or other reactive function on the fluorophore.
e) a fifth container means containing lyophilized PLL or amine reactive polymer in a stable form
f) a sixth container means containing the activating/coupling agent, such as HATU or EDC/NHS or EDC/HOBt or any other suitable activating/coupling agent combination in a stable form, more preferably dry form;
g) a seventh container means containing the activated small protecting/solubilizing moiety in a stable form for suitable coupling to the reactive amine function of polylysine or amine reactive polymer; and/or
h) a purification device, such as a desalting column or SEC column or ion exchange column.

### IV. PHARMACEUTICAL PREPARATIONS

An imaging probe according to the claims may be comprised in a pharmaceutical composition or a pharmaceutically acceptable excipient. Specific pharmaceutical compositions contemplated for delivery of an imaging probe include pharmaceutical compositions formulated for topical or systemic application (*e.g.*, oral, inhalational, intravenous, or intraperitoneal administration).

Pharmaceutical compositions of the present invention comprise an effective amount of one or more imaging probe or additional agent dissolved or dispersed in a pharmaceutically acceptable carrier. The phrases "pharmaceutical or pharmacologically acceptable" refers to molecular entities and compositions that do not produce an adverse, allergic or other untoward reaction when administered to an animal, such as, for example, a human, as appropriate. The preparation of an pharmaceutical composition that contains at least one imaging probe or additional active ingredient will be known to those of skill in the art in light of the present disclosure, as exemplified by Remington: The Science and Practice of Pharmacy 21st Edition, Lippincott Williams and Wilkins, Philadelphia, 2005. Moreover, for animal (*e.g.*, human) administration, it will be understood that preparations should meet sterility, pyrogenicity, general safety and purity standards as required by FDA Office of Biological Standards.

Pharmaceutical compositions may be applied systemically, orally or topically. Topical compositions include gels, creams, ointments, sprays, lotions, salves, sticks, soaps, powders, pessaries, aerosols, and other conventional pharmaceutical forms in the art. Ointments and creams may, for example, be formulated with an aqueous or oily base with the addition of suitable thickening and/or gelling agents. Lotions may be formulated with an aqueous or oily base and will, in general, also contain one or more emulsifying, dispersing, suspending, or thickening agent. Powders may be formed with the aid of any appropriate powder base. Drops may be formed with an aqueous or non-aqueous base containing, sometimes, one or more emulsifying, dispersing, or suspending agents.

Alternatively, the compositions may be provided in an adapted form for oral or parenteral administration, including intradermal, subcutaneous, intraperitoneal, or intravenous injection. Pharmaceutically acceptable formulations include plain or coated tablets, capsules, suspensions and solutions containing an imaging agent, optionally together with one or more inert conventional carriers and/or diluents, including corn starch, lactose, sucrose, microcrystalline cellulose, magnesium stearate, polyvinyl-pyrrolidone, citric acid, tartaric acid, water, water/ethanol, water/glycerole, water/sorbitol, water/polyethylenglycol, propylengycol, water/propyleneglycol/ethanol, water/polyethylenegycol/ethanol, stearylglycol, carboxymethylcellulose, phosphate buffer solution, or fatty substances such as hard fat or suitable mixtures thereof. Alternatively, the compounds according to the invention may be provided in liposomal formulations. Pharmaceutically acceptable liposomal formulations are well-known to persons skilled in the art and include phosphatidyl cholines, such as dimyristoyl phosphatidyl choline (DMPC), phosphatidyl choline (PC), dipalmitoyl phosphatidyl choline (DPPC), and distearoyl phosphatidyl choline (DSP), and phosphatidyl glycerols, including dimyristoyl phosphatidyl glycerol (DMPG) and egg phosphatidyl glycerol (EPG). Such liposomes may optionally include other phospholipids, *e.g.* phosphatidyl ethanolamine, phosphatic acid, phosphatidyl serine, phosphatidyl inositol, disaccarides or polysaccarides, including lactose, trehalose, maltose, maltotriose, palatinose, lactulose, or sucrose, *e.g.*, in a ratio of about 10-20 to about 0.5-6, respectively.

The concentrations of imaging agent administered to a subject (*e.g.*, a mammal such as a human patient) may vary depending upon the disease or tissue evaluated, the physical properties and/or pharmacokinetics of the imaging agent, the mode of administration, and the individual patient. The practitioner responsible for administration will, in any event, determine the concentration of active ingredient(s) in a composition and appropriate dose(s) for the individual subject. For topical application, *e.g.* concentration ranges from 0.05 to 50% (w/w), more preferentially from 0.1 to 20% may be used. Alternatively, for systemic application, doses of 0.05 mg/kg body weight to 1000 mg/kg body weight of fluorophore equivalents, more preferentially 0.1 to 100 mg/kg, may be used.

### EXAMPLES

### EXAMPLE 1

Preparation of a fluorophore-polymer conjugate comprised of a poly-L-lysine backbone with 15% loading of 5(6)-carboxylfluorescein via N-epsilon amide bonds: in a small vial fitted with a strong magnetic stirrer was dissolved PLL·HBr (8.0 mg, 3.23 × 10⁻⁴ mmol) in dry DMSO (0.9 mL) then was added DIPEA (3 equiv. per NH₂ side chain, 14.9 mg). This solution was stirred for 10 min. before adding drop wise and under vigorous stirring 5(6)carboxylfluorescein NHS ester (0.15 equiv. per NH₂ side chain) in DMSO (0.3 mL). The resulting solution was stirred in the dark for 2 h, then, the product, which selectively precipitates out of DMSO, was filtered and washed repeatedly with acetone to yield the desired product as an orange solid.

### EXAMPLE 2

Preparation of a fluorophore-polymer conjugate comprised of a poly-L-lysine backbone with 15% loading of 5(6)-carboxylfluorescein and 85% loading of moieties derived from 1-methylnicotinic acid via N-epsilon amide bonds: in a small vial fitted with a strong magnetic stirrer was dissolved PLL·HBr (8.0 mg, 3.23 × 10⁻⁴ mmol) in dry DMSO (0.9 mL) then was added DIPEA (3 equiv. per NH₂ side chain, 14.9 mg). This solution was stirred for 10 min. before adding drop wise and under vigorous stirring succinimidyl (1-methyl-3-pyridinio)formate iodide (0.30 equiv per epsilon NH₂ group in PLL) in DMSO (0.2 mL) and the solution stirred for 30 minutes, then was added under vigorous stirring 5(6)carboxylfluorescein NHS ester (0.15 equiv. per NH₂ side chain) in DMSO (0.3 mL). The resulting solution was stirred in the dark for 2 h, then an additional amount of N-succinimidyl (1-methyl-3-pyridinio)formate iodide (0.55 equiv per epsilon NH₂ group in PLL) in DMSO (0.4 mL) was added under vigorous stirring. The reaction solution was stirred for an additional 2 hours then excess solvent was removed by repeated extractions with diethyl ether (5X 10ml). The resulting solid was dissolved in water to make 4.0 mL of solution and the pH adjusted to 3 using trifluoroacetic acid. The aqueous phase was then filtered and the product purified by size exclusion chromatography using a sephacryl^{™} S-100 (Amersham Biosciences) column and 35:65:0.05 acetonitrile/water/TFA as eluent. The fraction containing the product was lyophilized to yield the desired product as a yellow fluffy solid.

### EXAMPLE 3

Preparation of a fluorophore-polymer conjugate comprised of a poly-L-lysine backbone with 15% loading of 5(6)-carboxylfluorescein and 85% loading of 5KDa mPEG chains via N-epsilon amide bonds: in a small vial fitted with a strong magnetic stirrer was dissolved PLL·HBr (8.0 mg, 3.23 ×10⁻⁴ mmol) in dry DMSO (0.9 mL) then was added DIPEA (3 equiv. per NH₂ side chain, 14.9 mg). This solution was stirred for 10 min. before adding drop wise and under vigorous stirring mPEG-NHS (Nektar Therapeutics) (0.10 equiv per epsilon NH₂ group in PLL) in DMSO (0.1 mL) and the solution stirred for 3 h. Then, 5(6)carboxylfluorescein NHS ester (0.15 equiv. per NH₂ side chain) in DMSO (0.3 mL) were added under stirring. The resulting solution was stirred in the dark for 3 h, then mPEG-NHS (Nektar Therapeutics) (0.75 equiv per epsilon NH₂ group in PLL) in DMSO (1.0 mL) was added under vigorous stirring. The reaction solution was stirred for an additional 8 hours then was diluted in water to make 4.0 mL of solution and the pH adjusted to 3 using trifluoroacetic acid. The reaction solution was then filtered and the product purified by size exclusion chromatography using a sephacryl^{™} S-100 (Amersham Biosciences) column and 35:65:0.05 acctonitrile/water/TFA as eluent. The fraction containing the product was lyophilized to yield the desired product as a yellow fluffy solid.

### EXAMPLE 4

Preparation of a fluorophore-polymer conjugate comprised of a poly-L-lysine backbone with 15% loading of 5(6)-carboxylfluorescein and 10% of moieties derived from p-nitrophenylbenzoic acid via N-epsilon amide bonds: in a small vial fitted with a strong magnetic stirrer was dissolved PLL·HBr (8.0 mg, 3.23 ×10⁻⁴ mmol) in dry DMSO (0.9 mL) then was added DIPEA (3 equiv. per NH₂ side chain of PLL, 14.9 mg). This solution was stirred for 10 min. before adding drop wise and under vigorous stirring N-succinimidyl p-nitrobenzoate (0.1 equiv. per NH₂ side chain of PLL) in DMSO (0.3 mL) and the resulting solution stirred for 1 h. At this time, 5(6)carboxylfluorescein NHS ester (0.15 equiv. per NH₂ side chain of PLL) in DMSO (0.3 mL). The resulting solution was stirred in the dark for 2 h, then, the product, which precipitated selectively from DMSO, was filtered and washed repeatedly with acetone. The product was obtained as an orange solid.

### EXAMPLE 5

Preparation of a fluorophore-polymer conjugate comprised of a poly-L-lysine backbone with 15% loading of 1-pyrenebutyric acid via N-epsilon amide bonds: in a small vial fitted with a strong magnetic stirrer was dissolved PLL·HBr (8.0 mg, 3.23 ×10⁻⁴ mmol) in dry DMSO (0.9 mL) then was added DIPEA (3 equiv. per NH₂ side chain, 14.9 mg). This solution was stirred for 10 min. before adding dropwise and under vigorous stirring 1-pyrenebutyric acid NHS ester (Aldrich) (0.15 equiv. per NH₂ side chain of PLL) in DMSO (0.4 mL). The resulting solution was stirred in the dark for 2 h, then, excess solvent was removed by repeated extractions with diethyl ether (5X 10ml). The resulting solid was dissolved in water to make 4.0 mL of solution and the pH adjusted to 3 using trifluoroacetic acid. The aqueous phase was then filtered and the product purified by size exclusion chromatography using a sephacryl^{™} S-100 (Amersham Biosciences) column and 35:65:0.05 acetonitrile/water/TFA as eluent. The fraction containing the product was lyophilized to yield the desired product as a colorless fluffy solid.

### EXAMPLE 6

Preparation of a fluorophore-polymer conjugate comprised of a poly-L-lysine backbone with 15% loading of 1-pyrenebutyric acid and 85% loading of moieties derived from 1-methylnicotinic acid via N-epsilon amide bonds: in a small vial fitted with a strong magnetic stirrer was dissolved PLL·HBr (8.0 mg, 3.23 ×10⁻⁴ mmol) in dry DMSO (0.9 mL) then was added DIPEA (3 equiv. per NH₂ side chain, 14.9 mg). This solution was stirred for 10 min. before adding dropwise and under vigorous stirring 1-pyrenebutyric acid NHS ester (Aldrich) (0.15 equiv. per NH₂ side chain of PLL) in DMSO (0.4 mL) and the solution stirred for 2 h, then succinimidyl (1-methyl-3-pyridinio)formate iodide (0.85 equiv per epsilon NH₂ group in PLL) in DMSO (0.6 mL) and the solution stirred for 2 h then excess solvent was removed by repeated extractions with diethyl ether (5X 10ml). The resulting solid was dissolved in water to make 4.0 mL of solution and the pH adjusted to 3 using trifluoroacetic acid. The aqueous phase was then filtered and the product purified by size exclusion chromatography using a sephacryl^{™} S-100 (Amersham Biosciences) column and 35:65:0.05 acetonitrile/water/TFA as eluent. The fraction containing the product was lyophilized to yield the desired product as a colorless fluffy solid.

### EXAMPLE 7

Preparation of a fluorophore-polymer conjugate comprised of a poly-L-lysine backbone with 15% loading of 1-pyrenebutyric acid and 85% loading of 5KDa mPEG chains via N-epsilon amide bonds: in a small vial fitted with a strong magnetic stirrer was dissolved PLL·HBr (8.0 mg, 3.23 × 10⁻⁴ mmol) in dry DMSO (0.9 mL) then was added DIPEA (3 equiv. per NH₂ side chain, 14.9 mg). This solution was stirred for 10 min. before adding dropwise and under vigorous stirring 1-pyrenebutyric acid NHS ester (Aldrich) (0.15 equiv) in DMSO (0.4 mL) and the solution stirred for 2h. Then, mPEG-NHS (Nektar Therapeutics) (0.85 equiv per epsilon NH₂ group in PLL) in DMSO (1.0 mL) and the solution stirred for 8 h, then it was diluted in water to make 4.0 mL of solution and the pH adjusted to 3 using trifluoroacetic acid. The reaction solution was then filtered and the product purified by size exclusion chromatography using a sephacryl^{™} S-100 (Amersham Biosciences) column and 35:65:0.05 acetonitrile/water/TFA as eluent. The fraction containing the product was lyophilized to yield the desired product as a colorless fluffy solid.

### EXAMPLE 8

Preparation of a fluorophore-polymer conjugate comprised of a poly-L-lysine backbone with 15% loading of 1-pyrenebutyric acid and 10% of moieties derived from p-nitrophenylbenzoic acid via N-epsilon amide bonds: in a small vial fitted with a strong magnetic stirrer was dissolved PLL·HBr (8.0 mg, 3.23 ×10⁻⁴ mmol) in dry DMSO (0.9 mL) then was added DIPEA (3 equiv. per NH₂ side chain of PLL, 14.9 mg). This solution was stirred for 10 min. before adding dropwise and under vigorous stirring *N*-succinimidyl *p-*nitrobenzoate (0.10 equiv. per NH₂ side chain of PLL) in DMSO (0.3 mL) and the resulting solution stirred for 1 h. At this time, 1-pyrenebutyric acid NHS ester (0.15 equiv. per NH₂ side chain of PLL) in DMSO (0.4 mL). The resulting solution was stirred in the dark for 2 h, then, excess solvent was removed by repeated extractions with diethyl ether (5X 10ml). The resulting solid was dissolved in water to make 4.0 mL of solution and the pH adjusted to 3 using trifluoroacetic acid. The aqueous phase was then filtered and the product purified by size exclusion chromatography using a sephacryl^{™} S-100 (Amersham Biosciences) column and 35:65:0.05 acetonitrile/water/TFA as eluent. The fraction containing the product was lyophilized to yield the desired product as a colorless fluffy solid.

### EXAMPLE 9

Preparation of a water soluble, non-quenched fluorophore-polymer conjugate comprised of a poly-L-lysine backbone with 0.5% loading of 1-pyrenebutyric acid via N-epsilon amide bonds: in a small vial fitted with a strong magnetic stirrer was dissolved PLL·HBr (8.0 mg, 3.23 × 10⁻⁴ mmol) in dry DMSO (0.9 mL) then was added DIPEA (3 equiv. per NH₂ side chain, 14.9 mg). This solution was stirred for 10 min. before adding dropwise and under vigorous stirring 1-pyrenebutyric acid NHS ester (Aldrich) (0.005 equiv. per NH₂ side chain of PLL) in DMSO (0.1 mL). The resulting solution was stirred in the dark for 2 h, then excess solvent was removed by repeated extractions with diethyl ether (5X 10ml). The resulting solid was dissolved in water to make 4.0 mL of solution and the pH adjusted to 3 using trifluoroacetic acid. The aqueous phase was then filtered and the product purified by size exclusion chromatography using a sephacryl^{™} S-100 (Amersham Biosciences) column and 35:65:0.05 acetonitrile/water/TFA as eluent. The fraction containing the product was lyophilized to yield the desired product as a colorless fluffy solid.

### EXAMPLE 10

Preparation of a fluorophore-polymer conjugate comprised of a poly-L-lysine backbone with 20% loading of a cathepsin D cleavable linker and 80% loading of 1-methylnicotinic acid through permissible epsilon *N*-amide bonds of the polylysine backbone: in a small vial fitted with a strong magnetic stirrer was dissolved PLL·HBr (8.0 mg, 3.23 ×10⁻⁴ mmol) and (NIR-667)-Gly-Pro-Ile-Cys(Et)-Phe-Phe-Arg-Leu-GlyOH·TFA (0.20 equiv per epsilon NH₂ group in PLL) in dry DMSO (1.2 mL) then was added DIPEA (4.0 equiv. per NH₂ side chain, 14.9 mg). This solution was stirred for 10 min. before adding HATU (Fluka) (0.3 equiv per epsilon NH₂ group in PLL) in dry DMSO (0.3 mL) and the mixture stirred for 10 h. At this time, *N*-succinimidyl (1-methyl-3-pyridinio)formate iodide (0.8 equiv per epsilon NH₂ group in PLL) in DMSO (0.6 mL) was added under vigorous stirring. The reaction solution was stirred for an additional 2 hours. At this time, excess solvent was removed by repeated extractions with diethyl ether (5X 10ml). The resulting solid was dissolved in water to make 4.0 mL of solution and the pH adjusted to 3 using trifluoroacetic acid. The aqueous phase was then filtered and the product purified by size exclusion chromatography using a sephacryl^{™} S-100 (Amersham Biosciences) column and 35:65:0.05 acetonitrile/water/TFA as eluent. The fraction containing the product was lyophilized to yield the desired product as a white fluffy solid.

For the preparation of (NIR-667)-Gly-Pro-Ile-Cys(Et)-Phe-Phe-Arg-Leu-GlyOH ·TFA: The peptide was manually assembled on the solid phase using the Fmoc strategy on a HN-Cys(Trt)-2-chlorotrityl resin (Bachem). Once the peptide reached the desired length (Boc-NH-Gly-Pro-Ile-Cys(Et)-Phe-Phe-Arg(Pbf)-Leu-Gly-2-chlorotrityl resin), it was cleaved/deprotected using a standard mixture of CH₂Cl₂/TFA/H₂O/EDT. The peptide was recovered in 80% yield and 90% purity after solvent evaporation and precipitation with diethyl ether. The terminal NH₂ function of the peptide was coupled chemoselectively to the near-infrared fluorophore using the following protocol: to a solution of the petide NH₂-Gly-Pro-Ile-Cys(Et)-Phe-Phe-Arg-Leu-GlyOH-2TFA (0.015 mmol) in dry DMSO (1.0 mL) was added DIPEA (3.0 equiv) then NIR-667 NHS (Fluka) (0.005 mmol, 3.6 mg) in DMSO (0.3 mL). The resulting solution was stirred for 6 hours and the product purified by reverse-phase HPLC on a C-18 column (Macherey-Nagel). Product was obtained as a white solid.

### EXAMPLE 11

Preparation of a fluorophore-polymer conjugate comprised of a poly-L-lysine backbone with 20% loading of a cathepsin D cleavable linker and 80% loading of mPEG (5 KDa) through permissible epsilon *N*-amide bonds of the polylysine backbone: in a small vial fitted with a strong magnetic stirrer was dissolved PLL·HBr (8.0 mg, 3.23 × 10⁻⁴ mmol) and (NIR-667)-Gly-Pro-Ile-Cys(Et)-Phe-Phe-Arg-Leu-GlyOH·TFA (0.20 equiv per epsilon NH₂ group in PLL) in dry DMSO (1.2 mL) then was added DIPEA (4.0 equiv. per NH₂ side chain, 14.9 mg). This solution was stirred for 10 min. before adding HATU (Fluka) (0.3 equiv per epsilon NH₂ group in PLL) in dry DMSO (0.3 mL) and the mixture stirred for 10 h. At this time, mPEG NHS ester (Nektar Therapeutics) (0.8 equiv per epsilo NH₂ group in PLL) in DMSO (0.6 mL) was added under vigorous stirring. The reaction solution was stirred for an additional 10 hours. At this time, the solution was diluted in water to make 4.0 mL of solution and the pH adjusted to 3 using trifluoroacetic acid. The solution was then filtered and the product purified by size exclusion chromatography using a sephacryl^{™} S-100 (Amersham Biosciences) column and 35:65:0.05 acetonitrile/water/TFA as eluent. The fraction containing the product was lyophilized to yield the desired product as a white fluffy solid.

### EXAMPLE 12

Preparation of a control non-activatible fluorophore-polymer conjugate comprised of a poly-L-lysine backbone with 20% loading of a via a D-configured cathepsin D non-cleavable linker and 80% loading of 1-methylnicotinic acid through permissible epsilon *N-*amide bonds of the polylysine backbone: in a small vial fitted with a strong magnetic stirrer was dissolved PLL·HBr (8.0 mg, 3.23 × 10⁻⁴ mmol) and (NIR-667)-Gly-Pro-(D)Ile-Cys(Et)-(D)Phe-Phe-(D)Arg-Leu-GlyOH·TFA (0.20 equiv. per epsilon NH₂ group in PLL) in dry DMSO (1.2 mL) then was added DIPEA (4.0 equiv. per NH₂ side chain, 14.9 mg). This solution was stirred for 10 min. before adding HATU (Fluka) (0.30 equiv per epsilon NH₂ group in PLL) in dry DMSO (0.3 mL) and the mixture stirred for 10 h. At this time, N-succinimidyl (1-methyl-3-pyridinio)formate iodide (0.8 equiv per epsilon NH₂ group in PLL) in DMSO (0.6 mL) was added under vigorous stirring. The reaction solution was stirred for an additional 2 hours. At this time, excess solvent was removed by repeated extractions with diethyl ether (5X 10ml). The resulting solid was dissolved in water to make 4.0 mL of solution and the pH adjusted to 3 using trifluoroacetic acid. The aqueous phase was then filtered and the product purified by size exclusion chromatography using a sephacryl^{™} S-100 (Amersham Biosciences) column and 35:65:0.05 acetonitrile/water/TFA as eluent. The fraction containing the product was lyophilized to yield the desired product as a white fluffy solid.

For the preparation of (NIR-667)-Gly-Pro-(D)Ile-Cys(Et)-(D)Phe-Phe-(D)Arg-Leu-GlyOH · TFA: The peptide was manually assembled on the solid phase in a similar fashion as illustrated in example 10. The terminal NH₂ function of the peptide was coupled chemoselectively to the near-infrared fluorophore using the same protocol illustrated in example 10.

### EXAMPLE 13

Preparation of a fluorophore-polymer conjugate comprised of a poly-L-lysine backbone with 20% loading of a cathepsin D cleavable linker with internal quencher (*p-*nitrobenzoic acid) and 80% loading of 1-methylnicotinic acid through permissible epsilon *N-*amide bonds of the polylysine backbone: in a small vial fitted with a strong magnetic stirrer was dissolved PLL·HBr (8.0 mg, 3.23 × 10⁻⁴ mmol) and (NIR-667)-Gly-Pro-Ile-Cys(Et)-Phe-Phe-Arg-Leu-Gly-Lys(*p*-nitrobenzamide)GlyOH ·TFA (0.20 equiv per epsilon NH₂ group in PLL) in dry DMSO (1.2 mL) then was added DIPEA (4.0 equiv. per NH₂ side chain, 14.9 mg). This solution was stirred for 10 min. before adding HATU (Fluka) (0.3 equiv per epsilon NH₂ group in PLL) in dry DMSO (0.3 mL) and the mixture stirred for 10 h. At this time, *N-*succinimidyl (1-methyl-3-pyridinio)formate iodide (0.8 equiv per epsilon NH₂ group in PLL) in DMSO (0.6 mL) was added under vigorous stirring. The reaction solution was stirred for an additional 2 hours. At this time, excess solvent was removed by repeated extractions with diethyl ether (5X 10ml). The resulting solid.was dissolved in water to make 4.0 mL of solution and the pH adjusted to 3 using trifluoroacetic acid. The aqueous phase was then filtered and the product purified by size exclusion chromatography using a sephacryl^{™} S-100 (Amersham Biosciences) column and 35:65:0.05 acetonitrile/water/TFA as eluent. The fraction containing the product was lyophilized to yield the desired product as a white fluffy solid.

For the preparation of (NIR-667)-Gly-Pro-Ile-Cys(Et)-Phe-Phe-Arg-Leu-Gly-Lys(*p-*nitrobenzamide)GlyOH _{·}TFA: The peptide was manually assembled on the solid phase using the Fmoc strategy on a HN-Cys(Trt)-2-chlorotrityl resin (Bachem). Once the peptide reached the desired length (Boc-NH-Gly-Pro-Ile-Cys(Et)-Phe-Phe-Arg(Pbf)-Leu-Gly-Lys(*p-*nitrobenzamide)-Gly-2-chlorotrityl resin), it was cleaved/deprotected using a standard mixture of CH₂Cl₂/TFA/H₂O/EDT. The peptide was recovered in 80% yield and 90% purity after solvent evaporation and precipitation with diethyl ether. The terminal NH₂ function of the peptide was coupled chemoselectively to the near-infrared fluorophore using the following protocol: to a solution of the petide NH₂-Gly-Pro-Ile-Cys(Et)-Phe-Phe-Arg-Leu-Gly-lys(*p*-nitrobenzamide)-GlyOH·2TFA (0.015 mmol) in dry DMSO (1.0 mL) was added DIPEA (3.0 equiv) then NIR-667 NHS (0.005 mmol, 3.6 mg) in DMSO (0.3 mL). The resulting solution was stirred for 6 hours and the product purified by reverse-phase HPLC on a C-18 column (Macherey-Nagel). Product was obtained as a white solid.

### EXAMPLE 14

In order to investigate the fluorescence quenching/activation behavior of fluorophore-PLL conjugates with respect to fluorophore the inventors first compared the use of two types of fluorophores: 1) hydrophilic fluorophore 5(6)carboxyfluorescein and 2) lipophilic fluorophore 1-pyrenebutyric acid (examples 1-10). Then, the inventors investigated the use of mPEG versus small charged 1-methylnicotinic acid chains as solubilizing/backbone protecting moieties. Then, the inventors investigated the use of nitro-substituted phenyl moieties like for example *p*-nitrobenzoic acid as additional quenching moieties.

**Preparation of stock solutions:** Stock solutions of each corresponding fluorescein conjugate were prepared in water by adjusting the optical density to 0.33 at 497nm. For pyrene conjugates, stock solutions in water were prepared having an optical density equal to 0.63 at 345nm.

**Fluorescence measurements for 5(6)-carboxyfluorescein containing probes:** 0.1 mL of the corresponding stock solution was diluted in D-PBS (1.0mL) and the fluorescence measured with a Fluoromax (λex= 492nm; λemission= 517nm). The results are summarized in Table 2.

**Fluorescence measurements for 1-pyrenebutyric acid containing probes:** 0.1 mL of the corresponding stock solution was diluted in D-PBS (1.0mL) and the fluorescence measured with a Fluoromax (λex= 344nm; λemission= 376nm). The results are summarized in Table 2.

**Enzymatic mediated fluorescence activation using trypsin:** 0.05 mL of the corresponding stock solution was diluted in 1.0 mL of D-PBS and the fluorescence measured at different time points at 37 °C after addition of either 0.05 mL of D-PBS or 0.05 mL of trypsin-EDTA solution (Sigma).

The results from this series of experiments revealed several surprising results. To begin with, it was clear that fluorescence quenching is far more complex than simply covalently binding a fluorophore to a polymer backbone (compare entries 1 and 5). Quenching efficiency is most effective when a lipophilic fluorophores like 1-pyrenebutyric acid (quenching factor of 10.5) are used instead of hydrophilic fluorophore like 5(6)-carboxyfluorescein (quenching factor of only 2.4). The optimal amphiphilic architecture attained with a combination between a lipophilic fluorophore and a hydrophilic polymer, such as PLL, ensures maximum fluorophore aggregation leading to excellent fluorescence quenching, while maintaining good water solubility. The use of 1-methylnicotinic acid moieties as additional quenchers/solubilizers had a remarkably positive impact on the probes. Thus, the fluorescence quenching factor for the fluorescein containing probe carrying 1-methylnicotinic acid moieties was 94 versus 2.4 for the probe having the same amount of fluorophore but lacking these additional charged moieties (Table 2, entries I and 2). Similarly, the fluorescence quenching factor for the pyrene containing probe carrying 1-methylnicotinic acid moieties was 1500 versus 10.5 for the probe lacking these additional charged moieties (Table 2, entries 5 and 6). Furthermore, although the use of 1-methylnicotinic acid moieties and mPEG both protected the backbone from enzymatic degradation/activation, the use of the former moiety is much more advantageous for probe construction since it dramatically increased fluorescence quenching while the latter decreased it (compare entries 2 with 3 and 6 with 7 of Table 2). The use of relatively small amounts of *p*-nitrobenzoic acid gave probes with surprisingly good quenching efficiencies (entries 4 and 8 of Table 2). These probes could then be activated by trypsin giving increases of 5.4 and 37 times for probes carrying fluorescein or pyrene, respectively.

### EXAMPLE 15

In order to investigate the fluorescence quenching/activation behavior of cathepsin D sensitive fluorophore-PLL conjugates (examples 9-11) *in vitro*, the inventors first investigated probe activation with cathepsin D, then carried out experiments with cathepsin D expressing cells.

Preparation of stock solutions: dissolve 1.0 mg of the corresponding conjugate in water to make 5.0 mL of solution. These stock solutions were stored at 5 °C in the dark.

Fluorescence measurements: Fluorescence measurements of equimolar solutions of each probe with respect to NIR-667 were performed with a Fluoromax (λex= 400nm; λemission= 667nm) and the results summarized in Table 2.

**Enzymatic mediated fluorescence activation using cathepsin D:** 0.05 mL of the corresponding stock solution was diluted in 1.0 mL of D-PBS and the fluorescence measured at different time points at 37 °C after addition of either 0.05 mL of D PBS or 0.05 mL of cathepsin D solution (Sigma).

The results from this series revealed that fluorescence activation by cathepsin D was most efficient for the probe carrying the 1-methylnicotinic acid moieties as backbone protecting units. The probe carrying mPEG or the scrambled, non-cleavable cathepsin D sequence were not activated by the enzyme. The surprising result that m-PEG chains inhibit the desired enzymatic activation of the probe, again indicate that small charged 1-methylnicotinic acid moieties are superior to mPEG in probe architecture.

### 1. Cells

The Cath D-1 cell line was prepared according to Liaudet *et al.* (1995). Cells were cultured in 24-well multiwell dishes using Dulbecco's Modified Minimum Essential Medium (DMEM) with Earle's salts containing 10% fetal calf serum (FCS), 100 U/ml penicillin 0.2 mg/ml streptomycin, 0.2 % glycine at 37°C in 5% CO₂, 95% air in a humidified atmosphere. After confluence, the cells were washed two times with HBSS.

### 2. Treatment and confocal fluorescence microscopy.

Cells were incubated with the NIR-PLL conjugates (examples 9 and 10) at 3-0.03µM concentrations. Incubation with the conjugates was performed for 60 minutes. The cells were rinsed with HBSS and incubated in the dark with DMEM for twenty-four hours. Confocal fluorescence microscopy was used to visualize probe activation within cells.

### 3. Results

The data indicates that the NIR-PLL activatible conjugate (example 9) indeed becomes considerably more fluorescent in the presence of cathepsin D positive cells. This fluorescence is greatly inhibited by using the non-activatable NIR-PLL conjugate (example 10). *In vitro* studies with cathepsin D expressing cells revealed activation of the probe within lysosomes.

**TABLE 2**

| *entry* | *solubilizer*/*quencher moiety* | *percent moiety* | *fluorophore* | *fluorescence queching factor at equimolar concentration* * | *fluorescence increase (times) upon enzymatic degradation* |
|---|---|---|---|---|---|
| 1 | none | - | fluorescein | 2.4 | 2.2 |
| 2 | 1-methylnicotinic acid | 85 | fluorescein | 94.0 | 1.6 |
| 3 | mPEG | 85 | fluorescein | 2.3 | 1.7 |
| 4 | *p*-nitrobenzoic acid | 10 | fluorescein | 6.8 | 5.4 |
| 5 | none | - | pyrene | 10.5 | 10.0 |
| 6 | 1-methylnicotinic acid | 85 | pyrene | 1500 | 1.5 |
| 7 | mPEG | 85 | pyrene | 6.2 | 1.7 |
| 8 | *p*-nitrobenzoic acid | 10 | pyrene | 46.7 | 37 |
| 9 | *1-methylnicotinic acid* | 25 | NIR-667 | 321 | 12 |
| 10 | *None* | 0 | NIR-667 | 137.7 | 12 |
| 11 | *1-methylnicotinic acid* | 86 | NIR-667 | 335 | 1 |
| 13 | *mPEG* | 25 | NIR-667 | 7.5 | 1.2 |

Data for 15% loaded fluorophore-PLL (DP=118.5) conjugates. *Equimolar concentration with respect to the fluorophore as determined by absorbance at 496nm for 5(6)carboxyfluorescein and at 345nm for 1-pyrenebutyric acid; quenching factor was determined by dividing fluorescence intensity of conjugate by that of the "free" non-quenched fluorophore. For fluorescein conjugates, comparison was made with the free fluorophore in D-PBS buffer, which was prepared by reacting 5(6)-carboxyfluorescein NHS ester with excess ammonia. The pyrene conjugates were compared to a non-quenched 0.5% loaded pyrene-PLL conjugate in D-PBS buffer.

### EXAMPLE 16

Five different urokinease plasminogen activator (uPA) sensitive probes have been prepared according to examples 10-12. N-uPA (L-conjugate) is a fluorescence reporter to which 20% of NIR667 was conjugated via an uPA sensitive peptide to polylysine and all other functions of the polymer were capped with N-methyl nicotinic acid. The corresponding D conjugate contains the same amino acid sequence made out of D-amino acids and was used as control. In S-uPA the functions remaining after conjugation of the uPA sensitive NIR peptide fragment were capped with succinic acid. mPEG-20-uPA is a copolymer of N-uPA (L-conjugate) and PEG (20kDa) and in mPEG-5000-uPA the functions remaining after conjugation of the uPA sensitive NIR peptide fragment were capped with mPEG (5 kDa). The fluorescence quenching of the probes was consistent with those in table 2 (Example 15).

Then, all conjugates were incubated with uPA at 37°C one hour and the percentage of totally cleaved NIR667 peptide conjugates were determined by using fluorimetric HPLC. The table below clearly demonstrates, that the use of small water solubilizing units on the polymeric backbone not only decreases the net fluorescence of the non-digested probe but also increase the total amount of released fluorophor peptide fragments from the probe as compared to the mPEG-5000-uPA.

**TABLE 3**

| Conjugate | % released |
|---|---|
| N-uPa (L-conjugate) | 33 |
| N-uPa (D-conjugate) | 0 |
| S-uPA | 11 |
| mPEG-20-uPA | 12 |
| mPEG-5000-uPA | 5 |

Influence of backbone substitution moiety on enzymatic cleavage: All conjugates were diluted with TRIS buffer (pH 7.4) to a concentration of 0.63 µM NIR667 equivalents. Digestion experiments were performed with 380nM urokinase (low molecular weight, 33kDa, from human urine, Calbiochem] during 60 minutes at 37°C.

### REFERENCES

U.S. Patent 4,649,151
U.S. Patent 4,866,168
U.S. Patent 4,883,790
U.S. Patent 4,920,143
U.S. Patent 5,002,962
U.S. Patent 5,028,621
U.S. Patent 5,093,349
U.S. Patent 5,166,197
U.S. Patent 5,171,747
U.S. Patent 5,171,749
U.S. Patent 5,173,504
U.S. Patent 5,198,460
U.S. Patent 5,438,071
U.S. Patent 6,147,195
U.S. Patent 6,592,847
US Application 2006/0275775

Agrawal and Zamecnik, Nucleic Acids Res., 18(18):5419-5423, 1990. Akhlynina et al., Cancer Res., 55(5):10.14-10.19, 1995.
Baldwin et al., Biochemistry, 29:5509-5515, 1990.
Beaucage, Methods Mol Biol., 20:33-61, 1993.
Biel, Laryngoscope, 108(9):1259-1268, 1998.
Bigelow et al., J. Microscopy, 215(1):24-33, 2004.
Birchler et al., J. Immunol. Methods, 231(1-2):239-248, 1999.
Birchler et al., Nat. Biotechnol., 17(10):984-988, 1999.
Boal et al., Nucleic Acids Res., 24(15):3115-3117, 1996.
Breinbauer and Koehn, Chem. Bio. Chem., 4(11):1147-1149, 2003.
Bressler and Bressler, Invest. Ophthalmol. Vis. Sci., 41(3):624-628, 2000.
Chen et al., FEBS Letters, 338:167-169, 1994.
Ching-Hsuan et al., Bioconjugate Chemistry, 10(5);892-896, 1999.
Coussens et al., Science, 295:2387-2392, 2002
Dc Rosa and Bentley, Pharm Res., 17(12):1447-1455, 2000.
Del Governatore et al., Cancer Res., 60(15):4200-4205, 2000.
Dougherty et al., J. Natl. Cancer Inst., 90(12):889-905, 1998.
Ell and Gossner, Cancer Res., 155:175-181, 2000.
Evans and Kargman, Current Pharm. Design, 10(6):627-634, 2004.
Ferkol et al., J. Clin. Invest., 92:2394-2400, 1993.
Fukuda et al., Int. J. Biochem. Cell Biology, 37(2):272-276, 2005.
Funovics et al., Analytical and Bioanalytical Chemistry, 377(6):956-963, 2003.
Funovics et al., Bioanalytical Chemistry, 377(6):956-963, 2003
Gomer et al., Cancer Res., 39(1):146-151, 1979.
Haimovici et al., Curr. Eye Res., 16(2):83-90, 1997..
Hamblin et al., J. Photochem. Photobiol. B, 26(1):45-56, 1994.
Hamblin et al., Photochem. Photobiol., 72, 2000.
Hochhaus and Larosse, Leukemia, 18(8):1321-1331, 2004.
Hoffman and Stayton, Fundl. Applns. Polymer Gels, 207,139-151, 2004.
Hsi et al., Drugs, 57(5):725-734, 1999.
James et al., Bioorg. Med. Chem. Lett., 9, 1999.
Koehn and Breinbauer, Angewandte Chemie, Intl. Ed., 43(24):3106-3116, 2004.
Kolb et al., Drug Discovery Today, 8(24):1128-1137, 2003.
Lange, In: Controlled drug delivery in photodynamic therapy and fluorescence-based diagnosis of cancer, Pogue and Mycek (eds.), Handbook of Fluorescence in Medicine Marcel Dekker, NY, 2002.
Levine et al., Comp. Biochem. Physiol., 72B:77-85, 1982.
Liaudet et al., Cell Growth Differ., 6(9):1045-1052, 1995.
Lindberg et al., Oncologica, 43(2):142-152, 2004.
Lopez et al., Advanced Drug Deliv. Rev., 56(1):77-94), 2004.
Mahmood and Weissleder, Molecular Cancer Therapeutics, 2(5):489-496, 2003.
Mattoussi et al., JALA, 9(1):28-32, 2004.
McGuire, Current Pharm. Design, 9(31):2593-2613, 2003.
McIntyre et al., Biochem. J., 377:617-628, 2004.
Means and Feeney, J. Food Biochem., 22(5):399-425, 1998.
Morris et al., Plant Molecular Biology, 24:673:77, 1994.
Nagae et al., J. Cardiovasc. Surg. (Torino), 39(6):709-715, 1998.
Nseyo, Urol. Clin. North Am., 19(3):591-599, 1992.
Ozaki and McLaughlin, Nuclei Acids Res., 20(19):5205-5214, 1992.
PCT Appln. WO 2004/004769
PCT Appln. WO 2005007631
PCT Appln. WO 2003082988
PCT Appln. WO 2002056670
PCT Appln. WO 2002000265
PCT Appln. WO 19958161
Pech et al., Baillieres Best Pract. Res. Clin. Gastroenterol., 15(2):267-284, 2001.
Pham et al., Bioconjugate Chemistry, 15(6):1403-1407, 2004.
Rau et al., Europ. J. Biochemistry, 268(11):3284-3295, 2001.
Rojanasakul et al., Pharmaceutical Res., 11(12):1731-1736, 1994.
Rosenkranz et al., Immunol. Cell Biol., 78(4):452-464,2000.
Seung-Cheol et al., Biochem. Biophysical Res. Comm., 327(4):979-984, 2005.
Sheski and Mathur, Cancer Control, 7(1):35-44, 2000.
Spitzer and Krumholz, Obstet. Gynecol. Clin. North Am., 18(3):649-659, 1991.
Sternberg and Dolphin, J. Clin. Laser Med. Surg., 11(5):233-241, 1993.
Tang and Agrawal, Nucleic Acids Res., 18(21):6461, 1990.
Veronese and Morpurgo, Farmaco, 54(8):497-516, 1999.
Vrouenraets et al., Cancer Res., 61(5):1970-1975, 2001.
Vrouenraets et al., Int. J. Cancer, 88(1):108-114, 2000.
Wainwright, J. Antimicrob Chemother., 42(1):13-28, 1998.
Wallace and Fraser, Amino Acids, 26(4):353-365, 2004.
Ward et al., Photochem. Photobiol., 35:803-808, 1982.
Wilbanks et al., J. Biol. Chem., 268:1226-1235, 1993.
Zhou et al., Proc. SPIE Intl. Soc. Optical Eng., 4955:505-512, 2003.
Coombs et al. J Biol Chem. 273: 4323-8, 1998.
Ke et al. J Biol Chem. 272: 16603-9, 1997.
Ke et al. J Biol Chem. 272: 20456-62, 1997b.
Rijkers et al. Thromb Res. 79: 491-9, 1995.
Backes et al. Nat Biotechnol. 18: 187-93, 2000.
Harris et al. Proc Natl Acad Sci U S A. 97: 7754-9, 2000.

## Claims

1. An imaging probe comprising a biocompatible hydrophilic polymer backbone conjugated to a plurality of hydrophobic fluorophores and a plurality of hydrophilic solubilizing moieties; wherein each solubilizing moiety is independently either 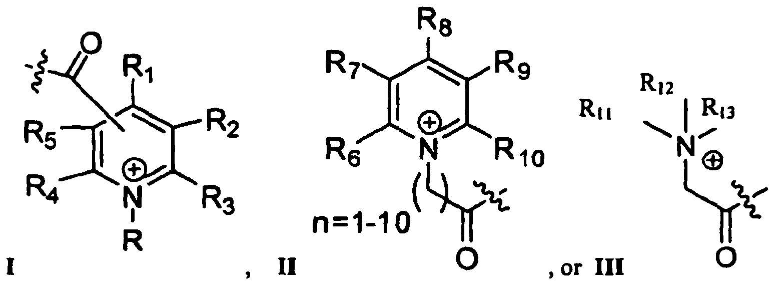 wherein R is independently hydrogen, a heteroatom-substituted or heteroatom-unsubstituted alkyl_{(C1-C10)}, aryl_{(C1-C10)}, or aralkyl_{(C2-C10)};
further wherein R₁, R₂, R₃, R₄ and R₅ are each independently hydrogen, hydroxy, amino, cyano, halo, nitro, mercapto, or a heteroatom-substituted or heteroatom-unsubstituted alkyl_{(C1-C10)}, aryl_{(C1-C10)}, aralkyl_{(C2-C10)}, acyl_{(C1-C10)}, alkoxy_{(C1-C10)}, aryloxy_{(C1-C10)}, aralkoxy_{(C2-C10)}, acyloxy_{(C1-C10)}, alkylamino_{(C1-C10)}, arylamino_{(C1-C10)}, aralkylamino_{(C2-C10)}, amido_{(C1-C10)}, or the carbonyl group that conjugates to the hydrophilic polymer backbone, provided that one of R₁, R₂, R₃, R₄ and R₅ is said carbonyl group that conjugates to the hydrophilic polymer backbone;
further wherein R₆, R₇, R₈, R₉ and R₁₀ are each independently hydrogen, hydroxy, amino, cyano, halo, nitro, mercapto, or a heteroatom-substituted or heteroatom-unsubstituted alkyl_{(C1-C10)}, aryl(_{C1-C10)}, aralkyl_{(C22-C10)}, acyl_{(C1-C10)}, alkoxy_{(C1-C10}, aryloxy_{(C1-C10)}, aralkoxy_{(C2-C10)}, acyloxy_{(C1-C10)}, alkylamino_{(C1-C10)}, arylamino_{(C1-C10)}, aralkylamino_{(C2-C10)}, or amido_{(C1-C10)};
further wherein R₁₁, R₁₂, and R₁₃, are each independently a heteroatom-substituted or heteroatom-unsubstituted alkyl_{(C1-C10)}, aryl_{(C1-C10)}, or aralkyl_{(C1-C10)};
further wherein the fluorophores comprise a near-infrared fluorophore, 1-pyrenebutyric acid (pyrene), NIR-667, NIR-641 N-succinimidyl ester, 11-((5-dimethylaminonaphthalene-1-sulfonyl)amino)undecanoic acid, cis-parinaric acid, BODIPY® 581/591, BODIPY® FL (505/511), BODIPY ® 530/550, BODIPY (C1-BODIPY 500/510 C12), C4-BODIPY 500/510 C9, C8-BODIPY 500/510 C5, a distyryl-boradiazaindacene, Dansyl (336/517), Marina Blue®, M12652 Marina Blue®, pacific blue TM, NBD (463/536), fluorescein (496/519), Oregon green® 488 (501/526), tetramethylrhodamine (540/566), lissamine rhodamine (560/581), lissamine rhodamine B sulfonyl chloride, texas red® (582/601), dansyl undecanoic acid, 6-(N-(7-nitrobenz-2-oxa-1,3-diazol-4-yl)amino)hexanoic acid, (4-fluoro-7-nitrobenz-2-oxa-1,3-diazole); (1-dodecyl,1'-(4-butyric acid NHS)-3,3,3',3'-tetramethylindocarbocyanine halide), phthalocyanines, (N-Ethyl-N-[5-(N-succininmidyloxycarbonyl)pentyl]-3, 3, 3, 3-tetramethyl-2), 2-indocarbocyanine chloride, 11-Chloro-1,1'-dipropyl-3,3,3',3'-tetramethyl-10,12-trimethyleneindatricarbocyanine iodide, NIR-664-N-succinimidyl ester, 2-[(E)-2-(2-chloro-3-{(E)-2-[3-ethyl-5-phenyl-1,3-benzoxazol-2(3H)-ylidene]ethylidene}-1-cyclopenten-1-yl)ethenyl]-3-ethyl-5-phenyl-1,3-benzoxazol-3-ium iodide, or 2-{2-chloro-3-[3-ethyl-1,3-benzothiazol-2(3H)-ylidene]-1-cyclopenten-1-yl}-3-ethyl-1,3-benzothiazol-3-ium perchlorate; and
further wherein at least some of said fluorophores are in fluorescence-quenching or self-quenching permissive positions which are separable by enzymatic cleavage.

2. The imaging probe of claim 1, wherein R is alkyl_{(C1-C6)} or n=1-6.

3. The imaging probe of claim 1, wherein the solubilizing moieties comprise 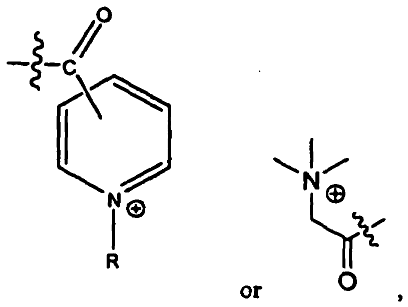 wherein R is an alkyl_{(C1-4)}.

4. The imaging probe of claim 3, wherein R is -CH₃.

5. The imaging probe of claim 1, wherein one or more of said fluorophores is conjugated to the polymer backbone via an enzyme-cleavable linker, and wherein cleavage of the enzyme-cleavable linker results in the release of at least one of said fluorophores from the polymer backbone.

6. The imaging probe of claim 1, wherein the polymer backbone is selected from the group consisting of polylysine, poly-L-lysine, poly-D-lysine, polyarginine, polyornithine, polyglutamic acid, and polypeptides.

7. The imaging probe of claim 6, wherein the polymer backbone is poly-L-lysine.

8. The imaging probe of claim 6, wherein fluorophore or the solubilizing moiety is conjugated to the polymer via an amide bond.

9. The imaging probe of claim 1, wherein the imaging probe further comprises a plurality of quenchers, a plurality of biocompatibilizing units, a targeting moiety, an antibody, or an antibody fragment.

10. The imaging probe of claim 1, wherein the imaging probe further comprises polyethylene glycol (PEG) or methoxypolyethylene glycol (mPEG).

11. The imaging probe of claim 1, wherein the imaging probe is comprised in a pharmaceutically acceptable excipient, a kit, or a container means.

12. The imaging probe of claim 1, wherein the polymer backbone is poly-L-lysine or polylysine, and wherein the solubilizing moieties are conjugated to the backbone via an amide bond, and wherein the solubilizing moieties comprise

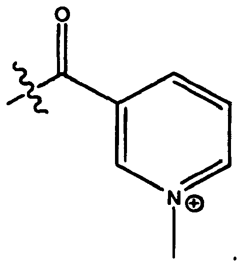

13. The imaging probe of any one of claims 1 to 12 for use in a method of *in vivo* photodetection or imaging, the method comprising:
i) administering the imaging probe to a subject;
ii) allowing a period of time to pass sufficient to allow for enzymatic activation of the imaging probe; and
iii) detecting the fluorescence of the fluorophores.

14. The imaging probe for use according to claim 13, wherein the subject is a mammal.

15. The imaging probe for use according to claim 13, wherein the method comprises a method for screening a putative protease inhibitor or a method of diagnosing or treating a disease; wherein the disease is a cell proliferative disease, cancer, breast cancer, an inflammatory disease, rheumathoid arthritis, a circulatory disease, atherosclerosis, a digestive disorder, ulcers, colon polyps, or is **characterized by** an activation of a proteolytic enzyme.

16. A method of *in vitro* photodetection or imaging comprising:
i) administering an imaging probe of any one of claims 1 to 12 to a sample;
ii) allowing a period of time to pass sufficient to allow for enzymatic activation of the imaging probe; and
iii) detecting the fluorescence of the fluorophores.

## Patentansprüche

1. Bildgebungssonde, umfassend ein biokompatibles hydrophiles Polymergerüst, das an eine Vielzahl hydrophober Fluorophore und eine Vielzahl hydrophiler löslichmachender Einheiten konjugiert ist; wobei jede löslichmachende Einheit unabhängig entweder 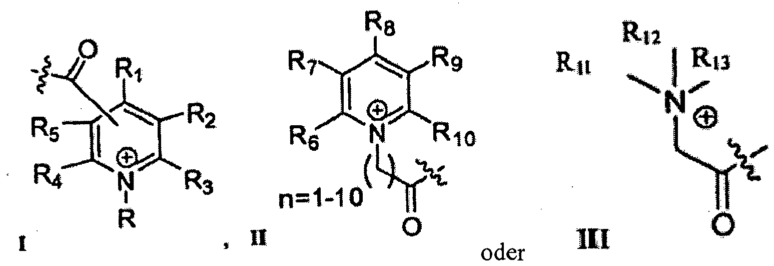 ist, wobei R unabhängig Wasserstoff, ein Heteroatom-substituiertes oder Heteroatom-unsubstituiertes (C₁-C₁₀)-Alkyl, (C₁-C₁₀)-Aryl oder (C₂-C₁₀)-Aralkyl ist;
wobei weiter R₁, R₂, R₃, R₄ und R₅ jeweils unabhängig Wasserstoff, Hydroxy, Amino, Cyano, Halogen, Nitro, Mercapto oder ein Heteroatom-substituiertes oder Heteroatom-unsubstituiertes (C₁-C₁₀)-Alkyl, (C₁-C₁₀)-Aryl, (C₂-C₁₀)-Aralkyl, (C₁-C₁₀)-Acyl, (C₁-C₁₀)-Alkoxy, (C₁-C₁₀)-Aryloxy, (C₂-C₁₀)-Arakoxy, (C₁-C₁₀)-Acyloxy, (C₁-C₁₀)-Alkylamino, (C₁-C₁₀)-Arylamino, (C₂-C₁₀)-Aralkylamino, (C₁-C₁₀)-Amido oder die Carbonylgruppe, die an das hydrophile Polymergerüst konjugiert ist, sind, mit der Maßgabe, dass eines von R₁, R₂, R₃, R₄ und R₅ die Carbonylgruppe ist, die an das hydrophile Polymergerüst konjugiert ist;
wobei weiter R₆, R₇, R₈, R₉ und R₁₀ jeweils unabhängig Wasserstoff, Hydroxy, Amino, Cyano, Halogen, Nitro, Mercapto oder ein Heteroatom-substituiertes oder Heteroatom-unsubstituiertes (C₁-C₁₀)-Alkyl, (C₁-C₁₀)-Aryl, (C₂-C₁₀)-Aralkyl, (C₁-C₁₀)-Acyl, (C₁-C₁₀)-Alkoxy, (C₁-C₁₀)-Aryloxy, (C₂-C₁₀)-Aralkoxy, (C₁-C₁₀)-Acyloxy, (C₁-C₁₀)-Alkylamino, (C₁-C₁₀)-Arylamino, (C₂-C₁₀)-Aralkylamino oder (C₁-C₁₀)-Amido sind; wobei weiter R₁₁, R₁₂ und R₁₃ jeweils unabhängig ein Heteroatom-substituiertes oder
Heteroatom-unsubstituiertes (C₁-C₁₀)-Alkyl, (C₁-C₁₀)-Aryl oder (C₂-C₁₀)-Aralkyl sind; wobei die Fluorophoren weiter ein Fluorophor im nahen Infrarotbereich, 1-Pyrenbuttersäure (Pyren), NIR-667, NIR-641 N-Succinimidylester, 11-((5-Dimethylaminonaphthalin-1-sulfonyl)amino)undecansäure, cis-Parinarsäure, BODIPY^{®} 581/591, BODIPY^{®} FL (505/511), BODIPY^{®} 530/550, BODIPY (C1-BODIPY 500/510 C12), C4-BODIPY 500/510 C9, C8-BODIPY 500/510 C5, ein Distyrylboradiazaindacen, Dansyl (336/517) Marina Blue^{®}, M12652 Marina Blue^{®}, Pacific blue TM, NBD (463/536), Fluorescein (496/519), Oregon green^{®} 488 (501/526), Tetramethylrhodamin (540/566), Lissaminrhodamin (560/581), Lissaminrhodamin B-sulfonylchlorid, Texas red^{®} (582/601), Dansylundecansäure, 6-(N-(7-Nitrobenz-2-oxa-1,3-diazol-4-yl)amino)hexansäure, (4-Fluor-7-nitrobenz-2-oxa-1,3-diazol), (1-Dodecyl,1'-(4-buttersäure-NHS)-3,3,3',3'-tetramethylindocarbocyaninhalogenid), Phthalocyanine, (N-Ethyl-N-[5-(N-succinimidyloxycarbonyl)pentyl]-3,3,3,3-tetramethyl-2), 2-indocarbocyaninchlorid, 11-Chlor-1,1'-dipropyl-3,3,3',3'-tetramethyl-10,12-trimethylenindatricarbocyaniniodid, NIR-664-N-Succinimidylester, 2-[(E)-2-(2-Chlor-3-{(E)-2-[3-ethyl-5-phenyl-1,3-benzoxazol-2(3H)-yliden]ethyliden}-1-cyclopenten-1-yl)ethenyl]-3-ethyl-5-phenyl-1,3-benzoxazol-3-iumiodid oder 2-{2-Chlor-3-[3-ethyl-1,3-benzothiazol-2(3H)-yliden]-1-cyclopenten-1-yl}-3-ethyl-1,3-benzothiazol-3-iumperchlorat umfassen; und
wobei weiter mindestens einige der Fluorophoren an Fluoreszenz-Löschung oder Selbstlöschung erlaubenden Positionen vorliegen, die durch enzymatische Spaltung trennbar sind.

2. Bildgebungssonde nach Anspruch 1, wobei R (C₁-C₆)-Alkyl ist oder n = 1-6.

3. Bildgebungssonde nach Anspruch 1, wobei die löslichmachenden
Einheiten 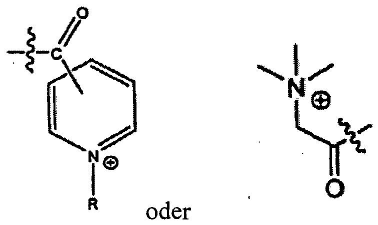 umfassen, wobei R ein (C₁-C₄)-Alkyl ist.

4. Bildgebungssonde nach Anspruch 3, wobei R -CH₃ ist.

5. Bildgebungssonde nach Anspruch 1, wobei eines oder mehrere der Fluorophoren über einen Enzym-spaltbaren Linker an das Polymergerüst konjugiert sind und wobei das Spalten des Enzym-spaltbaren Linkers die Freisetzung mindestens eines der Fluorophoren von dem Polymergerüst zur Folge hat.

6. Bildgebungssonde nach Anspruch 1, wobei das Polymergerüst aus der Gruppe bestehend aus Polylysin, Poly-L-lysin, Poly-D-lysin, Polyarginin, Polyornithin, Polyglutaminsäure und Polypeptiden ausgewählt ist.

7. Bildgebungssonde nach Anspruch 6, wobei das Polymergerüst Poly-L-lysin ist.

8. Bildgebungssonde nach Anspruch 6, wobei das Fluorophor oder die löslichmachende Einheit über eine Amidbindung an das Polymer konjugiert ist.

9. Bildgebungssonde nach Anspruch 1, wobei die Bildgebungssonde weiter eine Vielzahl von Quenchern, eine Vielzahl von Biokompatibilisierungseinheiten, eine Targeting-Einheit, einen Antikörper oder ein Antikörperfragment umfasst.

10. Bildgebungssonde nach Anspruch 1, wobei die Bildgebungssonde weiter Polyethylenglykol (PEG) oder Methoxypolyethylenglykol (mPEG) umfasst.

11. Bildgebungssonde nach Anspruch 1, wobei die Bildgebungssonde in einem pharmazeutisch verträglichen Exzipienten, einem Kit oder einer Behältervorrichtung beinhaltet ist.

12. Bildgebungssonde nach Anspruch 1, wobei das Polymergerüst Poly-L-lysin oder Polylysin ist und wobei die löslichmachenden Einheiten über eine Amidbindung an das Gerüst konjugiert sind und wobei die löslichmachenden Einheiten 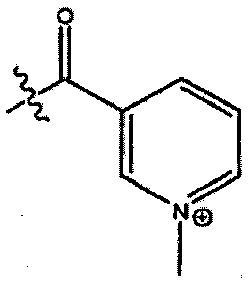 umfassen.

13. Bildgebungssonde nach einem der Ansprüche 1 bis 12 zur Verwendung in einem Verfahren der *in vivo*-Photodetektion oder -Bildgebung, wobei das Verfahren umfasst:
i) Verabreichen der Bildgebungssonde an ein Individuum;
ii) Verstreichenlassen eines ausreichenden Zeitraums, um eine enzymatische Aktivierung der Bildgebungssonde zu erlauben; und
iii) Nachweis der Fluoreszenz der Fluorophoren.

14. Bildgebungssonde zur Verwendung nach Anspruch 13, wobei das Individuum ein Säuger ist.

15. Bildgebungssonde zur Verwendung nach Anspruch 13, wobei das Verfahren ein Verfahren zum Screenen eines mutmaßlichen Proteaseinhibitors oder ein Verfahren zur Diagnose oder Behandlung einer Erkrankung umfasst; wobei die Erkrankung eine zellproliferative Erkrankung, Krebs, Brustkrebs, eine entzündliche Erkrankung, rheumatoide Arthritis, eine Kreislauferkrankung, Atherosklerose, eine Verdauungsstörung, Geschwüre, Dickdarmpolypen ist oder durch eine Aktivierung eines proteolytischen Enzyms gekennzeichnet ist.

16. Verfahren zur *in vitro*-Photodetektion oder -Bildgebung, umfassend:
i) Verabreichen einer Bildgebungssonde nach einem der Ansprüche 1 bis 12 an eine Probe;
ii) Verstreichenlassen eines ausreichenden Zeitraums, um eine enzymatische Aktivierung der Bildgebungssonde zu erlauben; und
iii) Nachweis der Fluoreszenz der Fluorophoren.

## Revendications

1. Sonde d'imagerie comprenant un squelette de polymère hydrophile biocompatible conjugué à une pluralité de fluorophores hydrophobes et à une pluralité de groupements solubilisants hydrophiles ; dans laquelle chaque groupement solubilisant est indépendamment l'un ou l'autre de 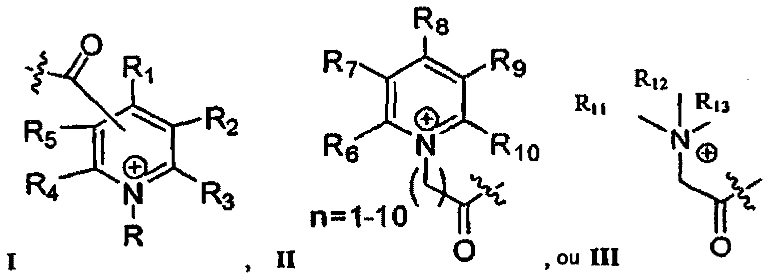 où R représente indépendamment un hydrogène ou un alkyle(en C₁ à C₁₀), un aryle(en C₁ à C₁₀) ou un aralkyle(en C₂ à C₁₀), substitué par un hétéroatome ou non substitué par un hétéroatome ;
où en outre R₁, R₂, R₃, R₄ et R₅ représentent chacun indépendamment un hydrogène, un hydroxy, un amino, un cyano, un halogéno, un nitro, un mercapto, ou un alkyle(en C₁ à C₁₀), un aryle(en C₁ à C₁₀), un aralkyle(en C₂ à C₁₀), un acyle(en C₁ à C₁₀), un alcoxy(en C₁ à C₁₀), un aryloxy(en C₁ à C₁₀), un aralcoxy(en C₂ à C₁₀), un acyloxy(en C₁ à C₁₀), un alkylamino(en C₁ à C₁₀), un arylamino(en C₁ à C₁₀), un aralkylamino(en C₂ à C₁₀), un amido(en C₁ à C₁₀), substitué par un hétéroatome ou non substitué par un hétéroatome, ou le groupe carbonyle qui est conjugué au squelette de polymère hydrophile, à condition que l'un de R₁, R₂, R₃, R₄ et R₅ soit ledit groupe carbonyle qui est conjugué au squelette de polymère hydrophile ;
où en outre R₆, R₇, R₈, R₉ et R₁₀ représentent chacun indépendamment un hydrogène, un hydroxy, un amino, un cyano, un halogéno, un nitro, un mercapto, ou un alkyle(en C₁ à C₁₀), un aryle(en C₁ à C₁₀), un aralkyle(en C₂ à C₁₀), un acyle(en C₁ à C₁₀), un alcoxy(en C₁ à C₁₀), un aryloxy(en C₁ à C₁₀), un aralcoxy(en C₂ à C₁₀), un acyloxy(en C₁ à C₁₀), un alkylamino(en C₁ à C₁₀), un arylamino(en C₁ à C₁₀), un aralkylamino(en C₂ à C₁₀) ou un amido(en C₁ à C₁₀), substitué par un hétéroatome ou non substitué par un hétéroatome ;
où en outre R₁₁, R₁₂ et R₁₃ représentent chacun indépendamment un alkyle(en C₁ à C₁₀), un aryle(en C₁ à C₁₀) ou un aralkyle(en C₂ à C₁₀), substitué par un hétéroatome ou non substitué par un hétéroatome ;
où en outre les fluorophores comprennent un fluorophore proche infrarouge, de l'acide 1-pyrènebutyrique (pyrène), du NIR-667, de l'ester N-succinimidylique NIR-641, de l'acide 11-((5-diméthylaminonaphtalène-1-sulfonyl)amino)undécanoïque, de l'acide cis-parinarique, du BODIPY® 581/591, du BODIPY® FL (505/511), du BODIPY® 530/550, du BODIPY(C1-BODIPY 500/510 C12), du C4-BODIPY 500/510 C9, du C8-BODIPY 500/510 C5, un distyryl-boradiazaindacène, du Dansyl (336/517), du Marina Blue®, du M12652 Marina Blute®, du pacific blue^{™}, du NBD (463/536), de la fluorescéine (496/519), de l'Oregon green® 488 (501/526), de la tétraméthylrhodamine (540/566), de la lissamine rhodamine (560/581), du chlorure de lissamine rhodamine B-sulfonyle, du texas red® (582/601), de l'acide dansyl undécanoïque, de l'acide 6-(N-(7-nitrobenz-2-oxa-1,3-diazol-4-yl)amino)hexanoïque, du (4-fluoro-7-nitrobenz-2-oxa-1,3-diazole), du (1-dodécyl,1'-(4-acide butyrique NHS)-halogénure de 3,3,3',3'-tétraméthylindocarbocyanine), des phtalocyanines, du (N-éthyl-N-[5-(N-succinimidyloxycarbonyl)pentyl]-3,3,3,3-tétraméthyle-2), du chlorure de 2-indocarbocyanine, de l'iodure de 11-chloro-1,1'-dipropyl-3,3,3',3'-tétraméthyl-10,12-triméthylèneindatricarbocyanine, de l'ester NIR-664-N-succinimidylique, de l'iodure 2-[(E)-2-(2-chloro-3-{(E)-2-[3-éthyl-5-phényl-1,3-benzoxazol-2(3H)-ylidéne]éthylidène}-1-cyclopentén-1-yl)éthényl]-3-éthyl-5-phényl-1,3-benzoxazol-3-ium ou du perchlorate de 2-{2-chloro-3-[3-éthyl-1,3-benzothiazol-2(3H)-ylidène]-1-cyclopenten-1-yl}-3-éthyl-1,3-benzothiazol-3-ium ; et
où en outre au moins certains desdits fluorophores sont dans des positions autorisant une extinction de fluorescence ou une auto-extinction qui sont séparables par clivage enzymatique.

2. Sonde d'imagerie selon la revendication 1, dans laquelle R est un alkyle(en C₁ à C₆) ou n = 1 à 6.

3. Sonde d'imagerie selon la revendication 1, dans laquelle les groupements solubilisants comprennent 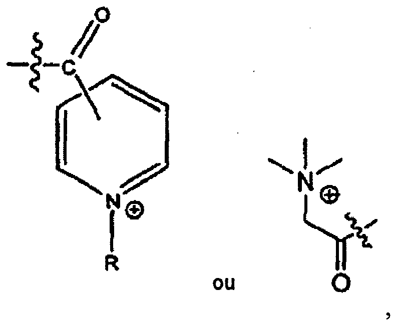 où R est un alkyle(en C₁ à C₄).

4. Sonde d'imagerie selon la revendication 3, dans laquelle R est -CH₃.

5. Sonde d'imagerie selon la revendication 1, dans laquelle un ou plusieurs desdits fluorophores sont conjugués au squelette de polymère par l'intermédiaire d'un coupleur pouvant être clivé par une enzyme, et dans laquelle le clivage du coupleur pouvant être clivé par une enzyme entraîne la libération d'au moins l'un desdits fluorophores du squelette de polymère.

6. Sonde d'imagerie selon la revendication 1, dans laquelle le squelette de polymère est choisi dans le groupe constitué par la polylysine, la poly-L-lysine, la poly-D-lysine, la polyarginine, la polyornithine, l'acide polyglutamique et des polypeptides.

7. Sonde d'imagerie selon la revendication 6, dans laquelle le squelette de polymère est de la poly-L-lysine.

8. Sonde d'imagerie selon la revendication 6, dans laquelle le fluorophore ou le groupement solubilisant est conjugué au polymère par l'intermédiaire d'une liaison amide.

9. Sonde d'imagerie selon la revendication 1, dans laquelle la sonde d'imagerie comprend en outre une pluralité d'extincteurs, une pluralité d'unités biocompatibilisantes, un groupement de ciblage, un anticorps ou un fragment d'anticorps.

10. Sonde d'imagerie selon la revendication 1, dans laquelle la sonde d'imagerie comprend en outre du polyéthylène glycol (PEG) ou du méthoxypolyéthylène glycol (mPEG).

11. Sonde d'imagerie selon la revendication 1, dans laquelle la sonde d'imagerie est contenue dans un excipient pharmaceutiquement acceptable, un kit ou des moyens de conditionnement.

12. Sonde d'imagerie selon la revendication 1, dans laquelle le squelette de polymère est de la poly-L-lysine ou de la polylysine, et dans laquelle les groupements solubilisants sont conjugués au squelette par l'intermédiaire d'une liaison amide, et dans laquelle les groupements solubilisants comprennent

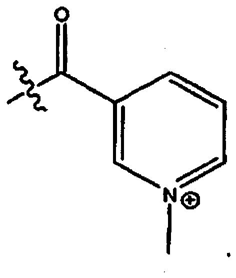

13. Sonde d'imagerie selon l'une quelconque des revendications 1 à 12, pour une utilisation dans un procédé de photodétection ou d'imagerie *in vivo*, le procédé comprenant :
i) l'administration de la sonde d'imagerie à un sujet ;
ii) l'écoulement d'un temps suffisant pour permettre l'activation enzymatique de la sonde d'imagerie ; et
iii) la détection de la fluorescence des fluorophores.

14. Sonde d'imagerie pour une utilisation selon la revendication 13, dans laquelle le sujet est un mammifère.

15. Sonde d'imagerie pour une utilisation selon la revendication 13, dans laquelle le procédé comprend un procédé de dosage d'un inhibiteur putatif de la protéase ou un procédé de diagnostic ou de traitement d'une maladie ; dans laquelle la maladie est une maladie à prolifération cellulaire, un cancer, un cancer du sein, une maladie inflammatoire, la polyarthrite rhumatoïde, une maladie circulatoire, l'athérosclérose, un trouble digestif, des ulcères, des polypes du côlon, ou est **caractérisée par** une activation d'une enzyme protéolytique.

16. Procédé de photodétection ou d'imagerie *in vitro* comprenant :
i) l'administration d'une sonde d'imagerie selon l'une quelconque des revendications 1 à 12 à un échantillon;
ii) l'écoulement d'un temps suffisant pour permettre l'activation enzymatique de la sonde d'imagerie ; et
iii) la détection de la fluorescence des fluorophores.
